# EUROPEAN PATENT SPECIFICATION

(11) **EP 2 612 921 B1**
(45) Date of publication and mention of the grant of the patent: **02.11.2016**
(21) Application number: 11821968.2
(22) Date of filing: 02.09.2011
(51) Int. Cl.: C07K 14/47, C12P 21/02, A61K 38/17, A61P 35/00

(54) **METHOD FOR PRODUCTION OF NOVEL DEGALACTOSYLATED GC GLOBULIN**
VERFAHREN ZUR HERSTELLUNG VON NEUEM DEGALACTOSYLIERTEM GC-GLOBULIN
PROCÉDÉ DE PRODUCTION D'UNE NOUVELLE GC GLOBULINE DÉGALACTOSYLÉE

(30) Priority: 03.09.2010 JP 2010197485
(43) Date of publication of application: 10.07.2013
(73) Proprietor: Morita Pharmaceutical Ind., Ltd., Tokyo 103-0022 (JP)
(72) Inventor: HORI Hitoshi, Tokushima-shi Tokushima 770-8506 (JP); UTO Yoshihiro, Tokushima-shi Tokushima 770-8506 (JP); TAKEUCHI Ryota, Nakagyo-ku, Kyoto-shi, Kyoto 604- 0805 (JP); NAKAGAWA Yoshinori, Soja-shi Okayama 719-1106 (JP); TERADA Hiroshi, Tokyo 162-8601 (JP); HIROTA Keiji, Tokyo 162-8601 (JP)
(74) Representative: Herzog, Fiesser & Partner Patentanwälte PartG mbB
(86) International application number: PCT/JP2011/070048
(87) International publication number: WO 2012/029954

(56) References cited:
- WO-A1-92/04459
- JP-A- H06 503 716
- JP-A- H06 510 908
- US-A1- 2006 014 143
- YAMAMOTO N ET AL: "VITAMIN D-3 BINDING PROTEIN GROUP-SPECIFIC COMPONENT IS A PRECURSOR FOR THE MACROPHAGE-ACTIVATING SIGNAL FACTOR FROM LYSOPHOSPHATIDYLCHOLINE-TREATED LYMPHOCYTES", PROCEEDINGS OF THE NATIONAL ACADEMY OF SCIENCES, NATIONAL ACADEMY OF SCIENCES, US, vol. 88, no. 19, 1 January 1991 (1991-01-01), pages 8539-8543, XP002145254, ISSN: 0027-8424, DOI: 10.1073/PNAS.88.19.8539
- NOBUTO YAMAMOTO ET AL: "Immunotherapy of HIV-infected patients with Gc protein-derived macrophage activating factor (GcMAF)", JOURNAL OF MEDICAL VIROLOGY, vol. 81, no. 1, 1 January 2009 (2009-01-01), pages 16-26, XP055010289, ISSN: 0146-6615, DOI: 10.1002/jmv.21376
- NOBUTO YAMAMOTO ET AL: "Immunotherapy for Prostate Cancer with Gc Protein-Derived Macrophage-Activating Factor, GcMAF", TRANSLATIONAL ONCOLOGY, NEOPLASIA PRESS, UNITED STATES, vol. 1, no. 2, 1 June 2008 (2008-06-01), pages 65-72, XP008154499, ISSN: 1944-7124, DOI: 10.1593/TLO.08106
- NOBUTO YAMAMOTO ET AL: "Immunotherapy of metastatic colorectal cancer with vitamin D-binding protein-derived macrophage-activating factor, GcMAF", CANCER IMMUNOLOGY, IMMUNOTHERAPY, SPRINGER, BERLIN, DE, vol. 57, no. 7, 6 December 2007 (2007-12-06), pages 1007-1016, XP019624360, ISSN: 1432-0851
- NOBUTO YAMAMOTO ET AL: "Immunotherapy of metastatic breast cancer patients with vitamin D-binding protein-derived macrophage activating factor (GcMAF)", INTERNATIONAL JOURNAL OF CANCER, vol. 122, no. 2, 15 January 2008 (2008-01-15), pages 461-467, XP055048283, ISSN: 0020-7136, DOI: 10.1002/ijc.23107
- BIN MOHAMAD SAHARUDDIN ET AL: "Preparation of Gc protein-derived macrophage activating factor (GcMAF) and its structural characterization and biological activities", ANTICANCER RESEARCH, INTERNATIONAL INSTITUTE OF ANTICANCER RESEARCH, GR, vol. 22, no. 6C, 1 November 2002 (2002-11-01), pages 4297-4300, XP009169604, ISSN: 0250-7005
- GOMME P T ET AL: "Therapeutic potential of vitamin D-binding protein", TRENDS IN BIOTECHNOLOGY, ELSEVIER PUBLICATIONS, CAMBRIDGE, GB, vol. 22, no. 7, 1 July 2004 (2004-07-01), pages 340-345, XP004520506, ISSN: 0167-7799, DOI: 10.1016/J.TIBTECH.2004.05.001
- NAGASAWA H ET AL.: "Gc Protein (Vitamin D-binding Protein): Gc Genotyping and GcMAF Precursor Activity", ANTICANCER RESEARCH, vol. 25, November 2005 (2005-11), pages 3689-3696, XP055094333,
- RAVNSBORG T ET AL: "The glycosylation and characterization of the candidate Gc macrophage activating factor", BIOCHIMICA ET BIOPHYSICA ACTA (BBA) - PROTEINS & PROTEOMICS, ELSEVIER, NETHERLANDS, vol. 1804, no. 4, 1 April 2010 (2010-04-01), pages 909-917, XP026921427, ISSN: 1570-9639, DOI: 10.1016/J.BBAPAP.2009.12.022 [retrieved on 2010-01-13]
- UTO YOSHIHIRO ET AL: "[beta]-Galactosidase treatment is a common first-stage modification of the three major subtypes of Gc protein to GcMAF.", ANTICANCER RESEARCH, vol. 32, no. 6, June 2012 (2012-06), pages 2359-2364, XP008166425, ISSN: 1791-7530
- HIROTA KEIJI ET AL: "Antitumor effect of degalactosylated gc-globulin on orthotopic grafted lung cancer in mice.", ANTICANCER RESEARCH, vol. 33, no. 7, July 2013 (2013-07), pages 2911-2915, XP008166427, ISSN: 1791-7530
- KUCHIIKE DAISUKE ET AL: "Degalactosylated/desialylated human serum containing GcMAF induces macrophage phagocytic activity and in vivo antitumor activity.", ANTICANCER RESEARCH, vol. 33, no. 7, July 2013 (2013-07), pages 2881-2885, XP008166426, ISSN: 1791-7530
- UTO Y. ET AL.: 'Effect of the Gc- derived Macrophage-activating Factor Precursor (preGcMAF) on Phagocytic Activation of Mouse Peritoneal Macrophages.' ANTICANCER RES. vol. 31, no. 7, July 2011, pages 2489 - 2492, XP055082396
- YAMAMOTO N. ET AL.: 'Conversion of vitamin D3 binding protein (group-specific component) to a macrophage activating factor by the stepwise action of beta-galactosidase of B cells and sialidase of T cells.' J. IMMUNOL. vol. 151, no. 5, 1993, pages 2794 - 2802, XP055082397
- CHRISTIANSEN M. ET AL.: 'Protein chemical characterization of Gc globulin (vitamin D-binding protein) isoforms; Gc-lf, Gc-ls and Gc-2.' BIOCHIM. BIOPHYS. ACTA vol. 1774, 2007, pages 481 - 492, XP022011915

### SEQUENCE LISTING

<110> The University of Tokushima Morita Pharmaceutical Ind., Ltd.
<120> A method of producing a novel Gc-globulin removced galactose
<130> PH-4949-PCT
<150> JP 2010-197485
   <151> 2010-09-03
<160> 3
<170> PatentIn version 3.5
<210> 1
   <211> 458
   <212> PRT
   <213> Homo sapiens
<400> 1
   Leu Glu Arg Gly Arg Asp Tyr Glu Lys Asn Lys Val Cys Lys Glu Phe
   1               5                   10                  15
   Ser His Leu Gly Lys Glu Asp Phe Thr Ser Leu Ser Leu Val Leu Tyr
               20                  25                  30
   Ser Arg Lys Phe Pro Ser Gly Thr Phe Glu Gln Val Ser Gln Leu Val
           35                  40                  45
   Lys Glu Val Val Ser Leu Thr Glu Ala Cys Cys Ala Glu Gly Ala Asp
       50                  55                  60
   Pro Asp Cys Tyr Asp Thr Arg Thr Ser Ala Leu Ser Ala Lys Ser Cys
   65                  70                  75                  80
   Glu Ser Asn Ser Pro Phe Pro Val His Pro Gly Thr Ala Glu Cys Cys
                   85                  90                  95
   Thr Lys Glu Gly Leu Glu Arg Lys Leu Cys Met Ala Ala Leu Lys His
               100                 105                 110
   Gln Pro Gln Glu Phe Pro Thr Tyr Val Glu Pro Thr Asn Asp Glu Ile
           115                 120                 125
   Cys Glu Ala Phe Arg Lys Asp Pro Lys Glu Tyr Ala Asn Gln Phe Met
       130                 135                 140
   Trp Glu Tyr Ser Thr Asn Tyr Glu Gln Ala Pro Leu Ser Leu Leu Val
   145                 150                 155                 160
   Ser Tyr Thr Lys Ser Tyr Leu Ser Met Val Gly Ser Cys Cys Thr Ser
                   165                 170                 175
   Ala Ser Pro Thr Val Cys Phe Leu Lys Glu Arg Leu Gln Leu Lys His
               180                 185                 190
   Leu Ser Leu Leu Thr Thr Leu Ser Asn Arg Val Cys Ser Gln Tyr Ala
           195                 200                 205
   Ala Tyr Gly Glu Lys Lys Ser Arg Leu Ser Asn Leu Ile Lys Leu Ala
       210                 215                 220
   Gln Lys Val Pro Thr Ala Asp Leu Glu Asp Val Leu Pro Leu Ala Glu
   225                 230                 235                 240
   Asp Ile Thr Asn Ile Leu Ser Lys Cys Cys Glu Ser Ala Ser Glu Asp
                   245                 250                 255
   Cys Met Ala Lys Glu Leu Pro Glu His Thr Val Lys Leu Cys Asp Asn
               260                 265                 270
   Leu Ser Thr Lys Asn Ser Lys Phe Glu Asp Cys Cys Gln Glu Lys Thr
           275                 280                 285
   Ala Met Asp Val Phe Val Cys Thr Tyr Phe Met Pro Ala Ala Gln Leu
       290                 295                 300
   Pro Glu Leu Pro Asp Val Arg Leu Pro Thr Asn Lys Asp Val Cys Asp
   305                 310                 315                 320
   Pro Gly Asn Thr Lys Val Met Asp Lys Tyr Thr Phe Glu Leu Ser Arg
                   325                 330                 335
   Arg Thr His Leu Pro Glu Val Phe Leu Ser Lys Val Leu Glu Pro Thr
               340                 345                 350
   Leu Lys Ser Leu Gly Glu Cys Cys Asp Val Glu Asp Ser Thr Thr Cys
           355                 360                 365
   Phe Asn Ala Lys Gly Pro Leu Leu Lys Lys Glu Leu Ser Ser Phe Ile
       370                 375                 380
   Asp Lys Gly Gln Glu Leu Cys Ala Asp Tyr Ser Glu Asn Thr Phe Thr
   385                 390                 395                 400
   Glu Tyr Lys Lys Lys Leu Ala Glu Arg Leu Lys Ala Lys Leu Pro Glu
                   405                 410                 415
   Ala Thr Pro Thr Glu Leu Ala Lys Leu Val Asn Lys Arg Ser Asp Phe
               420                 425                 430
   Ala Ser Asn Cys Cys Ser Ile Asn Ser Pro Pro Leu Tyr Cys Asp Ser
           435                 440                 445
   Glu Ile Asp Ala Glu Leu Lys Asn Ile Leu
       450                 455
<210> 2
   <211> 458
   <212> PRT
   <213> Homo sapiens
<400> 2
   Leu Glu Arg Gly Arg Asp Tyr Glu Lys Asn Lys Val Cys Lys Glu Phe
   1               5                   10                  15
   Ser His Leu Gly Lys Glu Asp Phe Thr Ser Leu Ser Leu Val Leu Tyr
               20                  25                  30
   Ser Arg Lys Phe Pro Ser Gly Thr Phe Glu Gln Val Ser Gln Leu Val
           35                  40                  45
   Lys Glu Val Val Ser Leu Thr Glu Ala Cys Cys Ala Glu Gly Ala Asp
       50                  55                  60
   Pro Asp Cys Tyr Asp Thr Arg Thr Ser Ala Leu Ser Ala Lys Ser Cys
   65                  70                  75                  80
   Glu Ser Asn Ser Pro Phe Pro Val His Pro Gly Thr Ala Glu Cys Cys
                   85                  90                  95
   Thr Lys Glu Gly Leu Glu Arg Lys Leu Cys Met Ala Ala Leu Lys His
               100                 105                 110
   Gln Pro Gln Glu Phe Pro Thr Tyr Val Glu Pro Thr Asn Asp Glu Ile
           115                 120                 125
   Cys Glu Ala Phe Arg Lys Asp Pro Lys Glu Tyr Ala Asn Gln Phe Met
       130                 135                 140
   Trp Glu Tyr Ser Thr Asn Tyr Gly Gln Ala Pro Leu Ser Leu Leu Val
   145                 150                 155                 160
   Ser Tyr Thr Lys Ser Tyr Leu Ser Met Val Gly Ser Cys Cys Thr Ser
                   165                 170                 175
   Ala Ser Pro Thr Val Cys Phe Leu Lys Glu Arg Leu Gln Leu Lys His
               180                 185                 190
   Leu Ser Leu Leu Thr Thr Leu Ser Asn Arg Val Cys Ser Gln Tyr Ala
           195                 200                 205
   Ala Tyr Gly Glu Lys Lys Ser Arg Leu Ser Asn Leu Ile Lys Leu Ala
       210                 215                 220
   Gln Lys Val Pro Thr Ala Asp Leu Glu Asp Val Leu Pro Leu Ala Glu
   225                 230                 235                 240
   Asp Ile Thr Asn Ile Leu Ser Lys Cys Cys Glu Ser Ala Ser Glu Asp
                   245                 250                 255
   Cys Met Ala Lys Glu Leu Pro Glu His Thr Val Lys Leu Cys Asp Asn
               260                 265                 270
   Leu Ser Thr Lys Asn Ser Lys Phe Glu Asp Cys Cys Gln Glu Lys Thr
           275                 280                 285
   Ala Met Asp Val Phe Val Cys Thr Tyr Phe Met Pro Ala Ala Gln Leu
       290                 295                 300
   Pro Glu Leu Pro Asp Val Arg Leu Pro Thr Asn Lys Asp Val Cys Asp
   305                 310                 315                 320
   Pro Gly Asn Thr Lys Val Met Asp Lys Tyr Thr Phe Glu Leu Ser Arg
                   325                 330                 335
   Arg Thr His Leu Pro Glu Val Phe Leu Ser Lys Val Leu Glu Pro Thr
               340                 345                 350
   Leu Lys Ser Leu Gly Glu Cys Cys Asp Val Glu Asp Ser Thr Thr Cys
           355                 360                 365
   Phe Asn Ala Lys Gly Pro Leu Leu Lys Lys Glu Leu Ser Ser Phe Ile
       370                 375                 380
   Asp Lys Gly Gln Glu Leu Cys Ala Asp Tyr Ser Glu Asn Thr Phe Thr
   385                 390                 395                 400
   Glu Tyr Lys Lys Lys Leu Ala Glu Arg Leu Lys Ala Lys Leu Pro Glu
                   405                 410                 415
   Ala Thr Pro Thr Glu Leu Ala Lys Leu Val Asn Lys Arg Ser Asp Phe
               420                 425                 430
   Ala Ser Asn Cys Cys Ser Ile Asn Ser Pro Pro Leu Tyr Cys Asp Ser
           435                 440                 445
   Glu Ile Asp Ala Glu Leu Lys Asn Ile Leu
       450                 455
<210> 3
   <211> 458
   <212> PRT
   <213> Homo sapiens
<400> 3
   Leu Glu Arg Gly Arg Asp Tyr Glu Lys Asn Lys Val Cys Lys Glu Phe
   1               5                   10                  15
   Ser His Leu Gly Lys Glu Asp Phe Thr Ser Leu Ser Leu Val Leu Tyr
               20                  25                  30
   Ser Arg Lys Phe Pro Ser Gly Thr Phe Glu Gln Val Ser Gln Leu Val
           35                  40                  45
   Lys Glu Val Val Ser Leu Thr Glu Ala Cys Cys Ala Glu Gly Ala Asp
       50                  55                  60
   Pro Asp Cys Tyr Asp Thr Arg Thr Ser Ala Leu Ser Ala Lys Ser Cys
   65                  70                  75                  80
   Glu Ser Asn Ser Pro Phe Pro Val His Pro Gly Thr Ala Glu Cys Cys
                   85                  90                  95
   Thr Lys Glu Gly Leu Glu Arg Lys Leu Cys Met Ala Ala Leu Lys His
               100                 105                 110
   Gln Pro Gln Glu Phe Pro Thr Tyr Val Glu Pro Thr Asn Asp Glu Ile
           115                 120                 125
   Cys Glu Ala Phe Arg Lys Asp Pro Lys Glu Tyr Ala Asn Gln Phe Met
       130                 135                 140
   Trp Glu Tyr Ser Thr Asn Tyr Gly Gln Ala Pro Leu Ser Leu Leu Val
   145                 150                 155                 160
   Ser Tyr Thr Lys Ser Tyr Leu Ser Met Val Gly Ser Cys Cys Thr Ser
                   165                 170                 175
   Ala Ser Pro Thr Val Cys Phe Leu Lys Glu Arg Leu Gln Leu Lys His
               180                 185                 190
   Leu Ser Leu Leu Thr Thr Leu Ser Asn Arg Val Cys Ser Gln Tyr Ala
           195                 200                 205
   Ala Tyr Gly Glu Lys Lys Ser Arg Leu Ser Asn Leu Ile Lys Leu Ala
       210                 215                 220
   Gln Lys Val Pro Thr Ala Asp Leu Glu Asp Val Leu Pro Leu Ala Glu
   225                 230                 235                 240
   Asp Ile Thr Asn Ile Leu Ser Lys Cys Cys Glu Ser Ala Ala Glu Asp
                   245                 250                 255
   Cys Met Ala Lys Gln Leu Gln Pro His Thr Val Lys Leu Cys Asp Asn
               260                 265                 270
   Leu Ser Thr Lys Asn Ser Lys Phe Glu Asp Cys Cys Gln Glu Lys Thr
           275                 280                 285
   Ala Met Asp Val Phe Val Cys Thr Tyr Phe Met Pro Ala Ala Gln Leu
       290                 295                 300
   Pro Glu Leu Pro Asp Val Glu Leu Pro Thr Asn Lys Asp Val Cys Asp
   305                 310                 315                 320
   Pro Gly Asn Thr Lys Val Met Asp Lys Tyr Thr Phe Glu Leu Ser Arg
                   325                 330                 335
   Arg Thr His Leu Pro Glu Val Phe Leu Ser Lys Val Leu Glu Pro Thr
               340                 345                 350
   Leu Lys Ser Leu Gly Glu Cys Cys Asp Val Glu Asp Ser Thr Thr Cys
           355                 360                 365
   Phe Asn Ala Lys Gly Pro Leu Leu Lys Lys Asp Leu Ser Ser Phe Ile
       370                 375                 380
   Asp Lys Gly Asn Glu Leu Cys Ala Asp Tyr Ser Glu Asn Thr Phe Thr
   385                 390                 395                 400
   Glu Tyr Lys Lys Lys Leu Ala Glu Arg Leu Lys Ala Lys Leu Pro Asp
                   405                 410                 415
   Ala Thr Pro Lys Glu Leu Ala Lys Leu Val Asn Leu Arg Ser Asp Phe
               420                 425                 430
   Ala Ser Asn Cys Cys Ser Ile Asn Ser Pro Pro Leu Tyr Cys Asn Ser
                   435                 440                 445
   Glu Ile Asn Ala Glu Leu Lys Asn Ile Leu
       450                 455

## Description

### Technical Field

The present invention relates to a method for producing degalactosylated Gc globulin (GcG) originating from blood plasma or serum and a pharmaceutical composition comprising, as an active ingredient, the degalactosylated GcG.

### Background Art

Animals have a natural healing ability that is spontaneously activated when they are injured. In such cases, Gc globulin, which is a glycoprotein in the serum, is deglycosylated by an inflammatory response and converted finally into GcMAF having N-acetylgalactosamine in its sugar chain in an organism. Such GcMAF activates macrophages. Specifically, Gc globulin serves as a GcMAF precursor (Yamamoto, N. et al., Proc. Natl. Acad. Sci., U.S.A., 88, 8539-8543, 1991; Yamamoto et al., J. Immunol. 151 (5), 2794-2802, 1993; WO 92/04459).

GcMAF has been reported to suppress or delay development of a variety of tumors through its macrophage activating activity and/or anti-angiogenic mechanism and exert growth inhibition activity on developed tumors (Bin Mohamad Saharuddin et al., Anticancer Research, 22, 4297-4300, 2002; Yamamoto, N. et al., Int. J. Cancer., 122 (2), 461-7, 2008; Yamamoto, N. et al., Transl. Oncol., 1 (2), 65-72, 2008; Yamamoto, N. et al., Cancer. Immunol. Immunother., 57 (7), 1007-16, 2008; Yamamoto, N. et al., Cancer Res., 57, 2187-92, 1997; Koga et al., Proc. Soc. Exp. Bil. Med., 220, 20-6, 1999; Korbelik et al., Br. J. Cancer. 75, 202-7, 1997; Yamamoto et al., Cancer Res., 56, 2827-31, 1996; Hashitani, S. et al., Official Journal of the Japanese Stomatological Society, 46 (5): 532, 1997; Kiser, O. et al., Neoplasia, 5: 32-40, 2003; Onizuka, S. et al., Pancreas 28(3): 317-319, 2003; and JP Patent Publication (Kohyo) No. 2003-532682 A).

As the antitumor effects of GcMAF have become elucidated, expectations for clinical applications thereof have been raised. In practice, however, therapeutic agents comprising GcMAF have not yet been developed.

GcMAF has been used for immunotherapy of HIV-infected patients (Yamamoto et al., Journal of Medical Virology, 81(1), 16-26, 2009).

For one reason, it is difficult to collect standardized Gc globulins as raw materials.

Human Gc globulin is considered to be provided with a sugar chain, such as galactose, sialic acid, mannose, or N-acetylgalactosamine, and there are at least 3 subtypes (1f, 1s, and 2). The subtype 1 is considered to comprise 3 sugars and the subtype 2 is considered to comprise 2 sugars. Since human serum contains a pair of Gc globulins each originating from either parent, an individual can have any of the following 6 subtype combinations: (1f, 1f), (1f, 1s), (1f, 2), (1s, 1s), (Is, 2), and (2, 2). More specifically, homogeneous subtypes (1f, 1f), (Is, Is), and (2, 2) and heterogeneous subtypes (If, Is), (1f, 2), and (1s, 2) exist.

The structure of the sugar chain associated with macrophage activation of each subset has been elucidated. In the case of the subtype If, threonines are present at positions 418 and 420, N-acetylgalactosamine is bound to threonine at either position 418 or 420, and galactose and sialic acid sugars are further bound to N-acetylgalactosamine. In the case of the subtype 1s, threonines are present at positions 418 and 420, N-acetylgalactosamine is bound to threonine at either position 418 or 420, and galactose and α-mannose are further bound to N-acetylgalactosamine. In the case of the subtype 2, threonine is present at position 418, N-acetylgalactosamine is bound thereto, and galactose is further bound thereto (Mohamad, S. B. et al., Anticancer Res., 23 (6A), 4451-7, 2003; and Nagasawa, H. et al., Anticancer Res., 24 (5C), 3361-6, 2004).

Of such globulin subtypes, deglycosylation reactions of the human serum Gc globulin of the subtype 1f-1f have been elucidated. Specifically, β-Galactosidase of B lymphocytes is activated when inflammation takes place in an organism, Gc globulin degalactosylated thereby is further desialized by sialidase of T cells, and GcMAF having only the N-acetylgalactosamine terminus is then generated.

As described above, human serum contains at least 3 types of Gc globulin subsets, and the process of deglycosylation has not yet been elucidated for all subsets. GcMAF mentioned above is produced with the exclusive use of the homogeneous subtype 1f-1f. Accordingly, no techniques for easily mass-producing GcMAF with the use of collected sera, plasma proteins, or Gc globulins (i.e., the standard technique) have been established, and development of a novel technique for producing GcMAF has been awaited in the art.

### Summary of the Invention

### Object to Be Attained by the Invention

The present invention provides a Gc globulin derivative that can be easily produced from collected sera, plasma proteins, or Gc globulins and can be used as GcMAF.

### Means for Attaining the Object

The present inventors have conducted concentrated studies in order to attain the above object. As a result, they discovered that degalactosylated GcG obtained by allowing β-galactosidase to react with Gc globulin can be used as GcMAF, regardless of Gc globulin subset, or such degalactosylated GcG can be easily converted into GcMAF via activation thereof *in vivo* or *in vitro*. This has led to the completion of the present invention.

Specifically, the present disclosure describes the following features.
[1] A method for producing a degalactosylated Gc globulin comprising allowing Gc globulin originating from blood plasma or serum to react with β-galactosidase.
[2] The method according to [1], wherein the Gc globulin is isolated or roughly purified from blood plasma or serum.
[3] The method according to [1] or [2], which further comprises a step of bringing the produced degalactosylated GcG into contact with lymphocytes or a culture supernatant of lymphocytes.
[4] A degalactosylated Gc globulin produced by the method according to any of [1] to [3].
[5] A pharmaceutical composition used for activating macrophages comprising one or a plurality of degalactosylated Gc globulins selected from the group consisting of (i) to (iv) below:
   (i) the degalactosylated Gc globulin of [4];
   (ii) a degalactosylated Gc globulin comprising the amino acid sequence as shown in SEQ ID NO: 1 and having a sugar chain structure in which N-acetylgalactosamine is bound to threonine at either position 418 or 420 of the amino acid sequence as shown in SEQ ID NO: 1 and sialic acid is further bound to N-acetylgalactosamine;
   (iii) a degalactosylated Gc globulin comprising the amino acid sequence as shown in SEQ ID NO: 2 and having a sugar chain structure in which N-acetylgalactosamine is bound to threonine at either position 418 or 420 of the amino acid sequence as shown in SEQ ID NO: 2 and α-mannose is further bound to N-acetylgalactosamine; and
   (iv) a degalactosylated Gc globulin comprising the amino acid sequence as shown in SEQ ID NO: 3 and having a sugar chain structure in which N-acetylgalactosamine is bound to threonine at position 418 of the amino acid sequence as shown in SEQ ID NO: 3.
[6] A pharmaceutical composition used for inhibition of angiogenesis comprising one or a plurality of degalactosylated Gc globulins selected from the group consisting of (i) to (iv) below:
   (i) the degalactosylated Gc globulin of [4];
   (ii) a degalactosylated Gc globulin comprising the amino acid sequence as shown in SEQ ID NO: 1 and having a sugar chain structure in which N-acetylgalactosamine is bound to threonine at either position 418 or 420 of the amino acid sequence as shown in SEQ ID NO: 1 and sialic acid is further bound to N-acetylgalactosamine;
   (iii) a degalactosylated Gc globulin comprising the amino acid sequence as shown in SEQ ID NO: 2 and having a sugar chain structure in which N-acetylgalactosamine is bound to threonine at either position 418 or 420 of the amino acid sequence as shown in SEQ ID NO: 2 and α-mannose is further bound to N-acetylgalactosamine; and
   (iv) a degalactosylated Gc globulin comprising the amino acid sequence as shown in SEQ ID NO: 3 and having a sugar chain structure in which N-acetylgalactosamine is bound to threonine at position 418 of the amino acid sequence as shown in SEQ ID NO: 3.
[7] A pharmaceutical composition used for treatment of cancer comprising one or a plurality of degalactosylated Gc globulins selected from the group consisting of (i) to (iv) below:
   (i) the degalactosylated Gc globulin of [4];
   (ii) a degalactosylated Gc globulin comprising the amino acid sequence as shown in SEQ ID NO: 1 and having a sugar chain structure in which N-acetylgalactosamine is bound to threonine at either position 418 or 420 of the amino acid sequence as shown in SEQ ID NO: 1 and sialic acid is further bound to N-acetylgalactosamine;
   (iii) a degalactosylated Gc globulin comprising the amino acid sequence as shown in SEQ ID NO: 2 and having a sugar chain structure in which N-acetylgalactosamine is bound to threonine at either position 418 or 420 of the amino acid sequence as shown in SEQ ID NO: 2 and α-mannose is further bound to N-acetylgalactosamine; and
   (iv) a degalactosylated Gc globulin comprising the amino acid sequence as shown in SEQ ID NO: 3 and having a sugar chain structure in which N-acetylgalactosamine is bound to threonine at position 418 of the amino acid sequence as shown in SEQ ID NO: 3.
[8] The pharmaceutical composition according to any of [5] to [7], wherein the degalactosylated Gc globulin is prepared from Gc globulin originating from blood plasma or serum of a subject to which the pharmaceutical composition is administered.
[9] A method for treatment of cancer comprising administering one or a plurality of degalactosylated Gc globulins selected from the group consisting of (i) to (iv) below to a cancer patient:
   (i) the degalactosylated Gc globulin of [4];
   (ii) a degalactosylated Gc globulin comprising the amino acid sequence as shown in SEQ ID NO: 1 and having a sugar chain structure in which N-acetylgalactosamine is bound to threonine at either position 418 or 420 of the amino acid sequence as shown in SEQ ID NO: 1 and sialic acid is further bound to N-acetylgalactosamine;
   (iii) a degalactosylated Gc globulin comprising the amino acid sequence as shown in SEQ ID NO: 2 and having a sugar chain structure in which N-acetylgalactosamine is bound to threonine at either position 418 or 420 of the amino acid sequence as shown in SEQ ID NO: 2 and α-mannose is further bound to N-acetylgalactosamine; and
   (iv) a degalactosylated Gc globulin comprising the amino acid sequence as shown in SEQ ID NO: 3 and having a sugar chain structure in which N-acetylgalactosamine is bound to threonine at position 418 of the amino acid sequence as shown in SEQ ID NO: 3.
[10] The method according to [9], wherein the degalactosylated Gc globulin is prepared from Gc globulin originating from blood plasma or serum of a cancer patient to which the degalactosylated GcG is administered.
[11] One or a plurality of degalactosylated Gc globulins used for treatment of cancer of a patient selected from the group consisting of (i) to (iv) below:
   (i) the degalactosylated Gc globulin of [4];
   (ii) a degalactosylated Gc globulin comprising the amino acid sequence as shown in SEQ ID NO: 1 and having a sugar chain structure in which N-acetylgalactosamine is bound to threonine at either position 418 or 420 of the amino acid sequence as shown in SEQ ID NO: 1 and sialic acid is further bound to N-acetylgalactosamine;
   (iii) a degalactosylated Gc globulin comprising the amino acid sequence as shown in SEQ ID NO: 2 and having a sugar chain structure in which N-acetylgalactosamine is bound to threonine at either position 418 or 420 of the amino acid sequence as shown in SEQ ID NO: 2 and α-mannose is further bound to N-acetylgalactosamine; and
   (iv) a degalactosylated Gc globulin comprising the amino acid sequence as shown in SEQ ID NO: 3 and having a sugar chain structure in which N-acetylgalactosamine is bound to threonine at position 418 of the amino acid sequence as shown in SEQ ID NO: 3.
[12] The degalactosylated Gc globulin according to [11], wherein the degalactosylated GcG is prepared from Gc globulin originating from blood plasma or serum of a cancer patient to which the degalactosylated GcG is administered.
[13] A degalactosylated Gc globulin comprising the amino acid sequence as shown in SEQ ID NO: 2 and having a sugar chain structure in which N-acetylgalactosamine is bound to threonine at either position 418 or 420 of the amino acid sequence as shown in SEQ ID NO: 2 and α-mannose is further bound to N-acetylgalactosamine; or
   a degalactosylated Gc globulin comprising the amino acid sequence as shown in SEQ ID NO: 3 and having a sugar chain structure in which N-acetylgalactosamine is bound to threonine at position 418 of the amino acid sequence as shown in SEQ ID NO: 3.

Specifically, the present invention pertains to a pharmaceutical composition comprising one or a plurality of human degalactosylated Gc globulins for use in healing of wounds or treating allergic diseases, autoallergic diseases, adverse reactions caused by therapeutic agents or angiogenic diseases, said one or a plurality of human degalactosylated Gc globulins being selected from the group consisting of (i) to (iii) below:
(i) the degalactosylated Gc globulin which is obtained by a treatment of Gc globulin of subtype 1f and/or subtype 1s derived from human blood plasma or serum with only β-galactosidase;
(ii) a human degalactosylated Gc globulin comprising the amino acid sequence as shown in SEQ ID NO: 1 and having a sugar chain structure in which N-acetylgalactosamine is bound to threonine at either position 418 or 420 of the amino acid sequence as shown in SEQ ID NO: 1 and sialic acid is further bound to N-acetylgalactosamine; and
(iii) a human degalactosylated Gc globulin comprising the amino acid sequence as shown in SEQ ID NO: 2 and having a sugar chain structure in which N-acetylgalactosamine is bound to threonine at either position 418 or 420 of the amino acid sequence as shown in SEQ ID NO: 2 and α-mannose is further bound to N-acetylgalactosamine.

Further, the invention relates to a pharmaceutical composition comprising one or a plurality of human degalactosylated Gc globulins for use in treating cancer, said one or a plurality of human degalactosylated Gc globulins being selected from the group consisting of (i) to (iii) below:
(i) the degalactosylated Gc globulin which is obtained by a treatment of Gc globulin of subtype 1f and/or subtype 1s derived from human blood plasma or serum with only β-galactosidase;
(ii) a human degalactosylated Gc globulin comprising the amino acid sequence as shown in SEQ ID NO: 1 and having a sugar chain structure in which N-acetylgalactosamine is bound to threonine at either position 418 or 420 of the amino acid sequence as shown in SEQ ID NO: 1 and sialic acid is further bound to N-acetylgalactosamine; and
(iii) a human degalactosylated Gc globulin comprising the amino acid sequence as shown in SEQ ID NO: 2 and having a sugar chain structure in which N-acetylgalactosamine is bound to threonine at either position 418 or 420 of the amino acid sequence as shown in SEQ ID NO: 2 and α-mannose is further bound to N-acetylgalactosamine.

The invention also pertains to a preparation comprising one or a plurality of degalactosylated Gc globulins for use in treating of cancer of a patient, selected from the group consisting of (i) to (iii) below:
(i) the degalactosylated Gc globulin which is obtained by a treatment of Gc globulin of subtype 1f and/or subtype 1s derived from human blood plasma or serum with only β-galactosidase;
(ii) a degalactosylated Gc globulin comprising the amino acid sequence as shown in SEQ ID NO: 1 and having a sugar chain structure in which N-acetylgalactosamine is bound to threonine at either position 418 or 420 of the amino acid sequence as shown in SEQ ID NO: 1 and sialic acid is further bound to N-acetylgalactosamine; and
(iii) a degalactosylated Gc globulin comprising the amino acid sequence as shown in SEQ ID NO: 2 and having a sugar chain structure in which N-acetylgalactosamine is bound to threonine at either position 418 or 420 of the amino acid sequence as shown in SEQ ID NO: 2 and α-mannose is further bound to N-acetylgalactosamine.

Moreover, the invention relates to a preparation comprising one or a plurality of human degalactosylated Gc globulins selected from the group consisting of (i) to (iii) below for use in healing of wound or treating allergic diseases, autoallergic diseases, adverse reactions caused by therapeutic agents or angiogenic diseases:
(i) the degalactosylated Gc globulin which is obtained by a treatment of Gc globulin of subtype 1f and/or subtype 1s derived from human blood plasma or serum with only β-galactosidase;
(ii) a human degalactosylated Gc globulin comprising the amino acid sequence as shown in SEQ ID NO: 1 and having a sugar chain structure in which N-acetylgalactosamine is bound to threonine at either position 418 or 420 of the amino acid sequence as shown in SEQ ID NO: 1 and sialic acid is further bound to N-acetylgalactosamine; and
(iii) a human degalactosylated Gc globulin comprising the amino acid sequence as shown in SEQ ID NO: 2 and having a sugar chain structure in which N-acetylgalactosamine is bound to threonine at either position 418 or 420 of the amino acid sequence as shown in SEQ ID NO: 2 and α-mannose is further bound to N-acetylgalactosamine.

### Effects of the Invention

The present invention can provide degalactosylated Gc globulin that can be easily produced regardless of Gc globulin subtype and used as GcMAF.

### Brief Description of the Drawings

Fig. 1 shows an image obtained by SDS-PAGE electrophoresis of a CBB-stained purified product (1f1fGcX). (P: positive control (Gc protein, Sigma); GcX: purified pre-GcMAF (1f1fGcX); M: marker)
Fig. 2 shows the results of Western blot analysis of purified pre-GcMAF (1f1fGcX) using a human anti-Gc globulin antibody. (Primary antibody: anti-human Gc globulin (10,000-fold diluted; the reaction was allowed to proceed at room temperature for 1 hour); secondary antibody: anti-rabbit HRP-IgG (10,000-fold diluted; the reaction was allowed to proceed at room temperature for 1 hour); exposure duration: 30 seconds; P: positive control (Gc protein, Sigma); GcX: purified pre-GcMAF (1f1fGcX); M: marker)
Fig. 3-1 shows the results of Western blot analysis of purified pre-GcMAF (1f1fGcX) using PNA lectin. (Primary antibody: PNA lectin (10,000-fold diluted; the reaction was allowed to proceed at room temperature for 1 hour); secondary antibody: streptavidin (10,000-fold diluted; the reaction was allowed to proceed at room temperature for 1 hour); exposure duration: 1 minute; Gc: Gc globulin; GcX: purified pre-GcMAF (1f1fGcX))
Fig. 3-2 shows the results of Western blot analysis of purified pre-GcMAF (1f1fGcX) using HPA lectin. (Primary antibody: HPA lectin (10,000-fold diluted; the reaction was allowed to proceed at room temperature for 1 hour); secondary antibody: streptavidin (10,000-fold diluted; the reaction was allowed to proceed at room temperature for 1 hour); exposure duration: 1 minute; MAF: purified GcMAF; GcX: purified pre-GcMAF (1f1fGcX))
Fig. 4-1 is a characteristic diagram showing the macrophage phagocytic capacity of purified pre-GcMAF (1f1fGcX).
Fig. 4-2 is a characteristic diagram showing the macrophage phagocytic capacity of purified pre-GcMAF (1f1fGcX) subjected to preliminary culture with non-adherent peritoneal cells.
Fig. 4-3 is a characteristic diagram showing the macrophage phagocytic capacity of a starting material; i.e., Gc globulin of the subtype 1f1f(1f1fGc). In the figure, the "*" symbol indicates the results of Experiment 2.
Fig. 5-1 is a characteristic diagram showing the macrophage phagocytic capacity of purified pre-GcMAF (1s1sGcX). In the figure, the "*" symbol indicates the results of Experiment 2.
Fig. 5-2 is a characteristic diagram showing the macrophage phagocytic capacity of a starting material; i.e., Gc globulin of the subtype 1s1s (1s1sGc). In the figure, the "*" symbol indicates the results of Experiment 2.
Fig. 6-1 is a characteristic diagram showing the macrophage phagocytic capacity of purified pre-GcMAF (22GcX). In the figure, the "*" symbol indicates the results of Experiment 2.
Fig. 6-2 is a characteristic diagram showing the macrophage phagocytic capacity of a starting material; i.e., Gc globulin of the subtype 22 (22Gc). In the figure, the "*" symbol indicates the results of Experiment 2.
Fig. 7 is a characteristic diagram showing the anti-tumor effects of purified pre-GcMAF (If1fGcX). Numerical values and error bars indicate the mean ± SEM values obtained in the experiment conducted at n = 3 or 4. In the figure, black regions in the error bars indicate the number of tumors grown to 2 mm or larger, and white regions indicate the number of tumors smaller than 2 mm. The "*" and "**" symbols in the figure each represent a significant decrease compared with the control group; i.e., (p < 0.05) and (p < 0.01), respectively, attained by the Bonferroni multiple comparison test.
Fig. 8 is a characteristic diagram showing the anti-tumor effects of various types of purified pre-GcMAF (22GcX, 1s1sGcX, and 1f1fGcX). Numerical values and error bars indicate the mean ± SEM values obtained in the experiment conducted at n = 3 or 4. In the figure, black regions in the error bars indicate the number of tumors grown to 2 mm or larger, and white regions indicate the number of tumors smaller than 2 mm. The "***" symbol in the figure represents a significant decrease compared with the control group (p < 0.001) attained by the Bonferroni multiple comparison test.

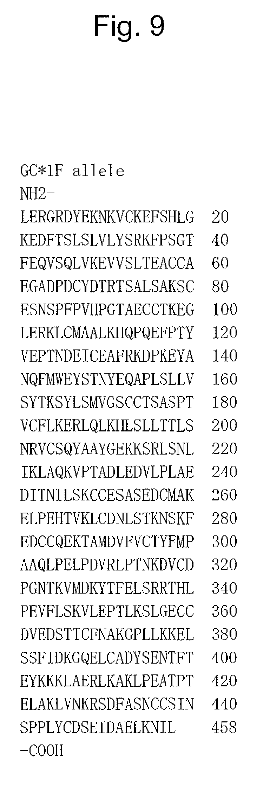

Fig. 9 shows the amino acid sequence of Gc globulin of the subtype 1f (SEQ ID NO: 1).

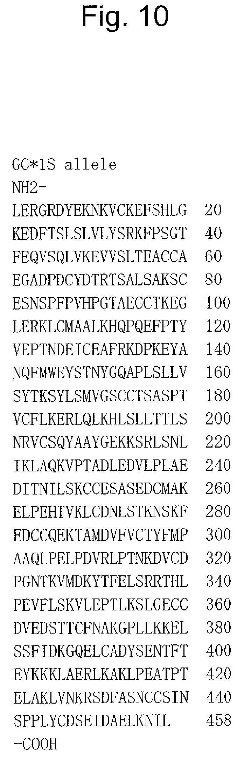

Fig. 10 shows the amino acid sequence of Gc globulin of the subtype 1s (SEQ ID NO: 2).

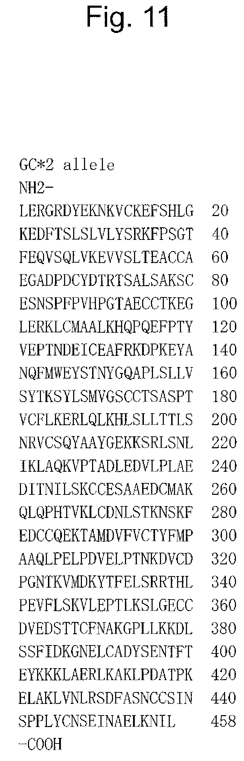

Fig. 11 shows the amino acid sequence of Gc globulin of the subtype 2 (SEQ ID NO: 3).

### Embodiments for Carrying out the Invention

The present disclosure describes a method for producing degalactosylated Gc globulin that can be easily produced regardless of Gc globulin subtype and used as GcMAF or easily converted into GcMAF (pre-GcMAF).

The method comprises allowing Gc globulin originating from blood plasma or serum to react with β-galactosidase.

Gc globulin that can be used in the present invention is derived from blood plasma, and preferably from blood serum. Gc globulin is contained in the serum at a density of approximately 300 to 500 mg/l, and the amount thereof is the 20th largest among serum proteins. With the use of blood plasma or serum, accordingly, large quantities of Gc globulins can be obtained efficiently.

Gc globulin may be contained in the blood plasma or serum; it may be in a roughly purified state, or it may be in an isolated state. In a "roughly purified state," Gc globulin is not substantially pure, but it contains impurities. The term "substantially pure" used herein refers to a degree of purity of at least 95%, and preferably at least 99%. In an "isolated state," the degree of purity is at least 95%, and preferably at least 99%. An isolated state is preferable.

Gc globulin can be isolated and purified from blood plasma or serum by a known method that is commonly employed for protein purification. Examples thereof include salting-out with ammonium sulfate, separation by precipitation using an organic solvent such as ethanol, methanol, or acetone, chromatography techniques, such as ion-exchange chromatography, isoelectric chromatography, gel filtration chromatography, hydrophobic chromatography, adsorption column chromatography, affinity chromatography using a substrate or antibody, and reversed-phase column chromatography, and filtration techniques, such as precision filtration, ultrafiltration, and reverse osmosis filtration. Such techniques can be performed alone or in adequate combinations of two or more. Preferably, the blood plasma or serum is applied to an affinity column, such as an actin-bound column, a 25-hydroxy-vitamin D₃-bound column, or an anti-Gc globulin antibody-bound column, so as to allow Gc globulin to specifically bind thereto, other proteins are liberated with an adequate wash solution, and Gc globulin is then eluted with an adequate eluate. Such procedure can also be performed by a batch method using 25-hydroxy-vitamin D₃-bound resin. Gc globulin is allowed to adsorb to the resin, followed by washing and elution with an acetate buffer or guanidine solution. The eluate can be concentrated by substituting buffer using Centricon or the like. 25-Hydroxy-vitamin D₃ is vitamin D₃ in which position 25 is hydroxylated, and the capacity thereof for specifically binding to Gc globulin is very high. With the use thereof, accordingly, Gc globulin can be separated and purified from blood plasma or serum with high purity. A method for obtaining Gc globulin from blood plasma via dialysis is known (e.g., JP Patent Publication (Kohyo) No. 2005-508892 A), and such method can be employed in the present invention.

Examples of Gc globulin that can be used in the present invention include Gc globulin that is artificially produced via genetic engineering (e.g., JP Patent Publication (Kohyo) No. H11-511962 A); i.e., Gc globulin obtained via recombinant expression in an adequate host cell with the use of a nucleic acid encoding Gc globulin. The amino acid sequence of Gc globulin is known and has been registered in the database of GenBank, or the like, and information of such sequences can be used.

β-Galactosidase that can be used in the present invention may originate from any organism. Alternatively, a recombinant enzyme obtained by recombinant expression in an adequate host cell using a nucleic acid encoding β-galactosidase can be obtained. β-Galactosidase can be in any state, such as a roughly purified, purified, or fixed state. Examples of roughly purified β-galactosidase include processed products from cell culture, such as extracts and lyophilized products. Commercially available β-galactosidase (e.g., from bovine liver, Grade III, Sigma) can also be used. In a fixed state, β-galactosidase is fixed to an adequate solid-phase substrate. Examples of materials constituting such solid-phase substrate include, but are not limited to, cellulose derivatives, such as cellulose and nitrocellulose, sepharose, agarose, metal, glass, ceramics, and resin. The configuration and material of a solid-phase substrate are not particularly limited.

Degalactosylation is carried out by allowing β-galactosidase to react with Gc globulin at 20°C to 60°C, preferably 35°C to 42°C, and more preferably 37.5°C for 0.5 to 5 hours, and preferably 0.5 to 2 hours. The reaction is carried out at pH 5 to pH 11, and preferably at a neutral level. Degalactosylation may be carried out by a batch method or a continuous method.

In every Gc globulin subtype, galactose is bound to N-acetylgalactosamine at the center of the sugar chain via an O-glycoside bond. Regardless of Gc globulin subtype, accordingly, degalactosylated GcG can be induced by allowing β-galactosidase to react with Gc globulin. The "degalactosylated GcG" is hereinafter referred to as "pre-GcMAF." Thus, "pre-GcMAF" can comprise a -(SA)-GalNAc sugar chain originating from the subtype If, a -(α-MAN)-GalNAc sugar chain originating from the subtype 1s, and/or a -GalNAc sugar chain originating from the subtype 2 in any combination depending on the subtype contained in the blood plasma or serum used as a material.

In the present invention, pre-GcMAF has any of the structures described below in accordance with the Gc globulin subtype.

Pre-GcMAF originating from Gc globulin of the subtype 1f comprises the amino acid sequence as shown in SEQ ID NO: 1 and has a sugar chain structure in which N-acetylgalactosamine is bound to threonine at either position 418 or 420 of the amino acid sequence as shown in SEQ ID NO: 1 and sialic acid is further bound to N-acetylgalactosamine.

Pre-GcMAF originating from Gc globulin of the subtype 1s comprises the amino acid sequence as shown in SEQ ID NO: 2 and has a sugar chain structure in which N-acetylgalactosamine is bound to threonine at either position 418 or 420 of the amino acid sequence as shown in SEQ ID NO: 2 and α-mannose is further bound to N-acetylgalactosamine.

Pre-GcMAF originating from Gc globulin of the subtype 2 comprises the amino acid sequence as shown in SEQ ID NO: 3 and has a sugar chain structure in which N-acetylgalactosamine is bound to threonine at position 418 of the amino acid sequence as shown in SEQ ID NO: 3.

In this description, the amino acid sequences as shown in SEQ ID NOs: 1, 2, and 3 include amino acid sequences having deletion, substitution, addition, or insertion of one to several amino acids in the amino acid sequences as shown in SEQ ID NOs: 1, 2, and 3 and encoding proteins having activities and functions of Gc globulin proteins. In such amino acid sequences, however, threonine at position 418 or 420 having the sugar chain structure as described above is conserved. Examples of "activities and functions of Gc globulin proteins" include those known in the art, such as the functions of a carrier protein that binds to vitamin D₃ and transports vitamin D₃ to target tissue, the functions of regulating polymerization of actin involved in regulation of cell division, configurational change, and movement (e.g., activity of accelerating depolymerization of F-actin and that of preventing repolymerization of G-actin), and the functions of macrophage activation upon conversion into GcMAF as described above. Such functions can be detected and assayed by a known technique. The range of "one to several" is not particularly limited. For example, it is preferably 1 to 10, more preferably 1 to 7, further preferably 1 to 5, and particularly preferably 1 to 3 or 1 or 2.

In this description, the amino acid sequences as shown in SEQ ID NOs: 1, 2, and 3, respectively, comprise or preferably consist of amino acid sequences having 90%, 95%, 99% or higher identity with such amino acid sequences determined by BLAST (the Basic Local Alignment Search Tool, the National Center for Biological Information, U.S.A.) using, for example, default parameters (i.e., initial parameters) and having activities and functions of Gc globulin proteins. In such amino acid sequences, however, threonine at position 418 or 420 having the sugar chain structure as described above is conserved.

The term "identity" used herein refers to the percentage of identical or similar amino acid residues relative to all the overlapping amino acid residues in the optimal alignment when two amino acid sequences are aligned with or without the introduction of a gap. Sequence identity can be determined by a method well-known in the art using sequence analysis software (e.g., a known algorithm such as BLAST or FASTA).

Even if pre-GcMAF contains a plurality of Gc globulin subtypes, it can uniformly be produced with the exclusive use of a single type of enzyme (i.e., β-galactosidase). Since it is not necessary to change enzyme types depending on subtypes, pre-GcMAF can be mass produced rapidly, and specialized pre-GcMAF can be produced in accordance with the needs of patients.

The produced pre-GcMAF may further be subjected to a purification process. Such purification process can be carried out by employing an adequate method for purification of Gc globulin, since the properties of pre-GcMAF are the same as those of Gc globulin.

As described in detail in the examples below, each pre-GcMAF produced in accordance with the present invention has different activity of macrophage activation depending on the Gc globulin subtype used. The term "activity of macrophage activation" used herein refers to activity of enhancing macrophage phagocytic capacity (Fc receptor-mediated phagocytic capacity, in particular), capacity for active oxygen production, or antigen presenting activity In this description, the term "macrophage phagocytic capacity" may occasionally be replaced with the term "macrophage phagocytic activity." These terms are interchangeable herein. Unlike GcMAF, pre-GcMAF originating from Gc globulin of the subtype 1f does not have activity of macrophage activation. When pre-GcMAF originating from Gc globulin of the subtype 1f is brought into contact with lymphocytes, and, in particular, T lymphocytes, it is converted into GcMAF, and it can then acquire activity of macrophage activation. By bringing pre-GcMAF originating from Gc globulin of the subtype 1f into contact with lymphocytes, activity of macrophage activation can be actually acquired in the supernatant. Alternatively, pre-GcMAF originating from Gc globulin of the subtype 1f may be administered into the body of a patient, brought into contact with lymphocytes in the body of a patient, and converted into GcMAF. As a result, GcMAF can acquire activity of macrophage activation. In contrast, pre-GcMAF originating from Gc globulin of the subtype 1 s and pre-GcMAF originating from Gc globulin of the subtype 2 have activity of macrophage activation. Such activity is maintained even when they are brought into contact with lymphocytes.

Pre-GcMAF thus prepared can be used as an active ingredient of a pharmaceutical composition used in treatment and prevention of a variety of diseases and disorders.

Pre-GcMAF contained in the pharmaceutical composition is prepared from blood plasma or serum. Alternatively, pre-GcMAF can be prepared from blood plasma or serum containing the Gc globulin subset identical to that of a subject to which the pharmaceutical composition is administered. More preferably, pre-GcMAF contained in the pharmaceutical composition is prepared from blood plasma or serum obtained from a subject to which the pharmaceutical composition is administered. With the use of blood plasma or serum obtained from a subject, the resulting pre-GcMAF, proteins contained in the blood plasma or serum, and/or derivatives thereof can be administered to the subject without the occurrence of problems, such as transmission of virus infections, production of irregular antibodies, onset of fever, or anaphylaxis. According to the present invention, the term "subject" refers to a human or non-human mammal, with a human being preferable.

It is possible for pre-GcMAF contained in the pharmaceutical composition to not be brought into contact with lymphocytes or a culture solution thereof. Alternatively, pre-GcMAF may be activated upon contact with the lymphocytes or a culture solution thereof (i.e., it maybe converted into GcMAF).

Examples of diseases and disorders that can be treated with the use of the aforementioned pharmaceutical composition include diseases and disorders that are known to be treated or may be treated by activation of macrophages or inhibition of angiogenesis. Examples of such diseases and disorders include, but are not limited to, wound healing, allergic diseases, autoallergic diseases, adverse reactions caused by therapeutic agents, cancer, and angiogenic diseases other than cancer. Examples of "cancer" include, but are not limited to, melanoma, metastasis, adenocarcinoma, sarcoma, thymoma, lymphoma, lung tumor, liver tumor, colon tumor, renal tumor, non-Hodgkin's lymphoma, Hodgkin's lymphoma, leukemia, uterine tumor, thoracic tumor, prostate tumor, renal tumor, ovarian tumor, spleen tumor, brain tumor, testicular tumor, bone tumor, muscle tumor, placental tumor, and gastrogenic tumor. Examples of "angiogenic diseases other than cancer" include, but are not limited to, diabetic retinopathy, retrolental fibroplasia, trachoma, neovascular glaucoma, psoriasis, immune inflammation (e.g., chronic rheumatoid arthritis, systemic lupus erythematosus, thyroiditis, Goodpasture's syndrome, generalized vasculitis, scleroderma, Sjogren's syndrome, sarcoidosis, primary biliary cirrhosis, and autoallergic diseases), non-immune inflammation, atherosclerosis, and excess wound healing.

The pharmaceutical composition of the present invention can be administered orally or parenterally. For example, it can be administered intravenously, intraarterially, topically by injection, intraperitoneally, intrathoracically, hypodermically, intramuscularly, sublingually, percutaneously, or intrarectally.

The pharmaceutical composition of the present invention can be prepared in an adequate dosage form in accordance with the route of administration. Specific examples of dosage forms include injection preparations, suspensions, emulsifiers, ointments, creams, tablets, capsules, granules, powders, pills, fine granules, troches, dosage forms for rectal administration, oleaginous suppositories, and water-soluble suppositories.

Such preparations can be produced in accordance with conventional techniques with the use of, for example, excipients, fillers, binders, wetting-out agents, disintegrators, surfactants, lubricants, dispersants, buffers, preservatives, solubilizers, antiseptics, colorants, flavoring agents, and stabilizers that are commonly used in the art.

Examples of excipients include lactose, fructose, glucose, corn starch, sorbit, crystalline cellulose, sterilized water, ethanol, glycerol, physiologic saline, and buffer. Examples of disintegrators include starch, sodium alginate, gelatin, calcium carbonate, potassium citrate, dextrin, magnesium carbonate, and synthetic magnesium silicate. Examples of binders include methylcellulose or a salt thereof, ethylcellulose, gum Arabic, gelatin, hydroxypropylcellulose, and polyvinylpyrrolidone. Examples of lubricants include talc, magnesium stearate, polyethylene glycol, and hydrogenated vegetable oil. Examples of stabilizers include amino acids such as arginine, histidine, lysine, and methionine, human serum albumin, gelatin, dextran 40, methylcellulose, sodium sulfite, and sodium metabisulfite. Examples of other additives include syrup, vaseline, glycerine, ethanol, propylene glycol, citric acid, sodium chloride, sodium nitrite, and sodium phosphate.

The pharmaceutical composition of the present invention can be aseptically produced by a conventional technique, such as filtration through a sterilization filter.

The dose of the pharmaceutical composition of the present invention can be determined in accordance with factors such as the age, body weight, or severity of diseases of a patient. The dose can be determined in such a manner that pre-GcMAF can be administered in an amount of 0.4 to 4,000 ng and preferably 20 to 2,000 ng per kg of the body weight in a single instance.

The effects of the pharmaceutical composition of the present invention *in vivo* can be assayed by comparing the conditions of remission, aggravation, or healing at a lesion before and after the administration of the pharmaceutical composition. For example, the effects of wound healing can be assayed by comparing the sizes of wounds and/or keloids before and after the administration of the pharmaceutical composition. Reduction in the sizes of wounds and/or keloids can be observed as a result of the administration of the pharmaceutical composition of the present invention. The antitumor effects of the pharmaceutical composition can be assayed by comparing the sizes of tumors and/or keloids before and after the administration of the pharmaceutical composition by a general means such as assays of dermal masses or the use of x-rays. Reduction in the sizes of dermal masses, tumors and/or keloids can be observed as a result of the administration of the pharmaceutical composition of the present invention. In the case of diabetic retinopathy, a retinal lesion may be diagnosed and evaluated before and after the administration of the pharmaceutical composition. Amelioration or absence of progression in the retinal lesion can be observed as a result of the administration of the pharmaceutical composition of the present invention. The effects of the pharmaceutical composition of the present invention can be assayed by a method that is commonly employed for inspection of effects attained by the administration of GcMAF.

The effects of pre-GcMAF *in vitro* can be evaluated based on the activity of macrophage activation. In general, the activity of GcMAF for macrophage activation can be inspected by extracting mouse peritoneal cells, performing preliminary culture in a serum-free medium, separating adherent cells from non-adherent cells, conducting culture of the adherent cells for 3 hours with the addition of GcMAF, and inspecting the phagocytic activity thereof (Nagasawa, H. et al. 2004, as above). Since pre-GcMAF originating from Gc globulin of the subtype 1s or 2 has the activity of macrophage activation as described above, the effects of pre-GcMAF can be evaluated by the method described above. However, pre-GcMAF originating from Gc globulin of the subtype 1f does not have the activity of macrophage activation. Thus, evaluation cannot be conducted by the method described above. Accordingly, non-adherent cells are cultured for 2 hours with the addition of pre-GcMAF, the adherent cells are cultured for 3 hours with the addition of the resulting culture solution, and the phagocytic activity of the cultured cells is then assayed. Thus, the effects of pre-GcMAF can be evaluated.

The present invention further describes the method for treatment and prevention of the diseases and disorders described above with the use of the pharmaceutical composition of the present invention.

In the present invention, pre-GcMAF is Gc globulin prepared from serum Gc globulin by selectively eliminating galactose. Pre-GcMAF originating from the subtype 1s or 2 can be used in the same manner as in the case of GcMAF. Pre-GcMAF originating from the subtype 1f can be efficiently and easily converted into GcMAF *in vivo* and can be used in the same manner as in the case of GcMAF. As in the case of GcMAF, accordingly, activity of macrophage activation, activity of angiogenesis inhibition, and, further, reinforcement of the resistance or healing ability of a patient can be expected with the administration of pre-GcMAF to a patient.

Hereafter, the present invention is described in greater detail with reference to examples.

### Examples

### Example 1: Synthesis of pre-GcMAF from purified Gc globulin and fractionation

### (I) Collection of Gc globulin from serum

A blood sample (20 ml) obtained from a human having Gc globulin of the subtype 1f1f was allowed to stand at room temperature for 30 minutes and then centrifuged at 4°C and 3,000 rpm for 10 minutes to obtain 10.3 ml of blood serum.

STE buffer (3.5 ml, pH 7.4, 6.05 g of Tris·HCl, 0.56 g of EDTA·2NA, 8.77 g of NaCl, 1,000 ml of SDW, and 1 ml of Triton-100) was added to 3.5 ml of the blood serum sample to bring the total amount to 7.0 ml, and the resultant was allowed to flow through an affinity column (10 mm × 80 mm) containing 25(OH)VD₃-bound beads (synthesized in the laboratory of Professor Hori at the Institute of Technology and Science, University of Tokushima) at a rate of 0.4 ml/min. Further, STE buffer was allowed to flow through the column for 90 minutes, 6 M guanidinium hydrochloride was allowed to flow therethrough at a rate of 2.0 ml/min, and about 20 ml of the eluted solution containing proteins was collected. The collected solution was injected into a dialysis bag with one end thereof closed with a clip, the other end thereof was closed with a clip, a float was applied thereto, the resultant was allowed to float in 4 liters of 5 mM phosphate buffer (sodium phosphate buffer (SPB)), and dialysis was then carried out. The dialysate solution was exchanged with fresh solution 90 minutes, 180 minutes, and 270 minutes after the initiation of dialysis. The third step of dialysis was carried out overnight.

After dialysis, the solution was collected and allowed to flow through the hydroxyapatite column (BioRad, Bio-Scale™ Mini CHT Type III, 40 µM Cartridge, Catalog # 732-4332, Lot No. B012409B) at a rate of 0.2 ml/min. Thereafter, 5 mM phosphate buffer (SPB) was allowed to flow therethrough at a rate of 2.0 ml/min to wash the column. When the baseline was stabilized, gradient elution was carried out with 200 mM phosphate buffer (SPB, pH 7.0), and approximately 9 ml of a Gc globulin-containing solution was collected. The collected solution was applied to Centricon (Millipore, Lot No. R0DA20931, 30,000 MWCO), and centrifugation was carried out in a centrifuge provided with a collection cup and an angle rotor at 3,900 G for 10 minutes to obtain purified Gc globulin (742.67 µg/300 µl).

Purified Gc globulin was also obtained from a human having Gc globulin of the subtype 1s1s or 22 in the same manner as described above.

### (II) Synthesis of pre-GcMAF from Gc globulin and concentration

β-galactosidase (10 mU/µl, 25 µl, from bovine liver, Grade III, Sigma, Lot No. 54H7025, G1875) was added to 25 µg of purified Gc globulin, and 165 µl of 100 mM SPB (pH 7.0) was added thereto to bring the total amount of the solution to 200 µl. The resultant was incubated at 37.5°C for 1 hour. The reaction solution was transferred to a 2.0-ml Eppendorf tube containing 1.0 g of 25(OH)VD₃-bound beads (synthesized in the laboratory of Professor Hori at the Institute of Technology and Science, University of Tokushima), the tube was subjected to shaking at 4°C for 1 hour, and centrifugation was carried out at 4°C and 13,000 rpm for 2 minutes to remove the supernatant. STE buffer (0.5 ml) was added to the precipitate, the resultant was subjected to shaking for 60 seconds, and centrifugation was carried out at 4°C and 13,000 rpm for 2 minutes to remove the supernatant. This procedure was repeated 3 times. Subsequently, an eluate (i.e., 0.4 ml of 6 M guanidinium hydrochloride) was added, the resultant was subjected to shaking for 60 seconds, and centrifugation was carried out at 13,000 rpm for 2 minutes. This procedure was repeated 3 times, and the resulting solutions were mixed to obtain an extract containing pre-GcMAF.

The extract containing pre-GcMAF thus obtained was applied to Microcon (Millipore, Lot No. R9DN95311, 10,000 MWCO) and centrifuged in a centrifuge with a collection cup at 4°C and 13,500 rpm for 10 minutes, the solution was exchanged with 10 mM SPB buffer, and 20 µl of purified pre-GcMAF (1f1fGcX) was obtained. The obtained purified pre-GcMAF was used in the examples below.

### Example 2: Examination of properties of purified pre-GcMAF (1f1fGcX)

### (I) Protein visualization by SDS-PAGE of purified pre-GcMAF (1f1fGcX) (CBB staining), Western blot analysis using human anti-Gc globulin, and HPLC analysis

Protein concentration of purified pre-GcMAF (1f1fGcX) was assayed using the bicinchoninic acid (BCA) protein assay kit (Pierce, Reagent A: Lot No. HH106101, Prod. # 23223; Reagent B: Lot No. CE49183, Prod. # 23224) and found to be 17.1 µg/20 µl.

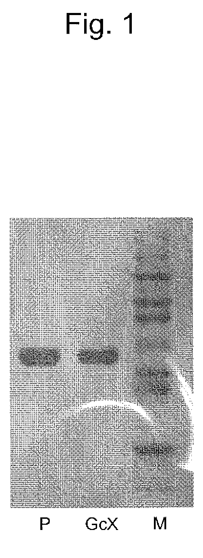

Proteins separated by SDS-PAGE were visualized via Coomassie Brilliant Blue (CBB) staining. As a result, a single dark band was observed at around the molecular weight of 56 kDa (Fig. 1).

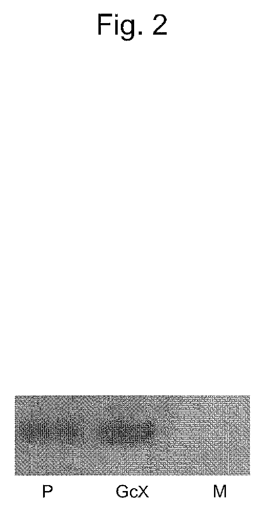

Since this band exhibited a positive reaction to Western blot analysis using the human anti-Gc globulin antibody, it was confirmed to be a Gc globulin derivative (Fig. 2).

Since purified pre-GcMAF (1f1fGcX) exhibited a single protein band, protein analysis was carried out by HPLC (280 nm), and the peak thereof was found to be consistent with the peak of Gc globulin (Sigma) at a retention time of 12.554 min.

### (II) Western blot analysis of purified pre-GcMAF (1f1fGcX) using PNA lectin and HPA lectin

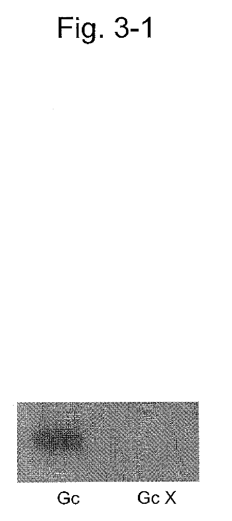

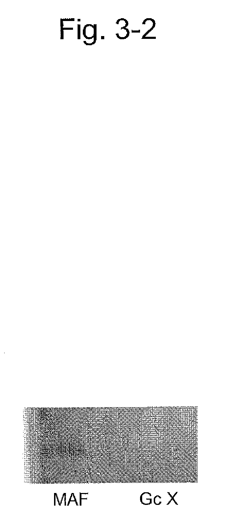

In order to inspect the sugar chain structure of purified pre-GcMAF (1f1fGcX), a target band (a dark band observed at around 56 kDa) was subjected to Western blot analysis using PNA lectin. As a result, purified Gc globulin was stained with PNA lectin; however, purified pre-GcMAF (1f1fGcX) was not stained with PNA lectin (Fig. 3-1). As a result of Western blot analysis of the target band using HPA lectin, purified GcMAF (synthesized in the laboratory of Professor Hori at the Institute of Technology and Science, University of Tokushima) was stained with HPA lectin; however, the band of purified pre-GcMAF (1f1fGcX) observed at around 56 kDa was not stained with HPA lectin (Fig. 3-2).

The above results demonstrate that Gc globulin has the Gal-(SA)-GalNAc sugar chain, GcMAF has the GalNAc sugar chain, and the sugar chain structure of purified pre-GcMAF (1f1fGcX) is not consistent with either thereof.

### Example 3-1: Influence of purified pre-GcMAF (1f1fGcX) on macrophage phagocytic activity (in vitro)

According to a phagocytosis test of sheep red blood cells (SRBC) using Fc receptors of mouse peritoneal macrophages *in vitro*, Gc globulin is known to have no effects on macrophage phagocytic activity, but GcMAF is known to enhance macrophage phagocytic activity (N. Yamamoto, N. P. Willett, and D. D. Lindsay, Inflammation, 1994, 18 (3): 311-322; N. Yamamoto, S. Homma, and I. Millman, J. Immunol., 1991, 147: 273-280). Based thereon, the influence of purified pre-GcMAF originating from Gc globulin of the subtype 1f1f (1f1fGcX) on macrophage phagocytic activity was inspected.

The influence of purified pre-GcMAF on macrophage phagocytic activity was evaluated in the manner described below.

### <Experiment 1>

Into the peritoneal cavity of an 8-week-old ICR mouse (female), 10 ml of PBS was injected, and the cells mixed in the peritoneal cavity were extracted and centrifuged at 4°C and 1,000 rpm for 15 minutes. The density of the collected cells was adjusted to 1.0 x 10⁶ cells/ml in RPMI 1640 medium, and the cell-containing solution was seeded on a plate containing a cover slip sunk therein in an amount of 500 µl each to adjust the density to 5.0 x 10⁵ cells/well. RPMI medium (500 µl) was added to the cells, and the cells were subjected to preliminary culture at 37.5°C for 1 hour to fix macrophages to the cover slip. Thereafter, the supernatant was removed, the macrophage-adhered layer was washed, fresh RPMI medium was added, and culture was conducted at 37°C for 15 hours.

To the resulting macrophage layer, 10 ng of purified pre-GcMAF, 10 ng of purified GcMAF (synthesized in the laboratory of Professor Hori at the Institute of Technology and Science, University of Tokushima), or 1 µg of LPS (Sigma) was added, and culture was conducted at 37°C for 3 hours. Subsequently, 0.5% SRBC coated with IgG was added to the macrophages, the macrophages were allowed to ingest SRBC for 90 minutes, and the macrophages were fixed and subjected to Giemsa staining. Thereafter, the ingested SRBCs were counted under a microscope, and the ingestion index was determined in order to evaluate the macrophage phagocytic activity.

### <Experiment 2>

The supernatant resulting from the preliminary culture conducted in Experiment 1 was recovered, 10 ng of purified pre-GcMAF was added to about 1 ml of the recovered supernatant, and culture was conducted at 37°C for 1 hour. Thereafter, the culture product was centrifuged to remove non-adherent cells contained in the medium, the solution was added to the macrophage layer prepared in the same manner as in Experiment 1 as a culture solution, and culture was conducted at 37°C for 3 hours. Subsequently, 0.5% SRBC coated with IgG was added to the macrophages, the macrophages were allowed to ingest SRBC for 90 minutes, and the cells were fixed and subjected to Giemsa staining. Thereafter, the ingested SRBCs were counted under a microscope, and the ingestion index was determined in order to evaluate the macrophage phagocytic activity.

As a control sample, the supernatant of the peritoneal fluid extracted from the mouse peritoneal cavity was used (equivalent to the supernatant resulting from the preliminary culture conducted in Experiment 1).

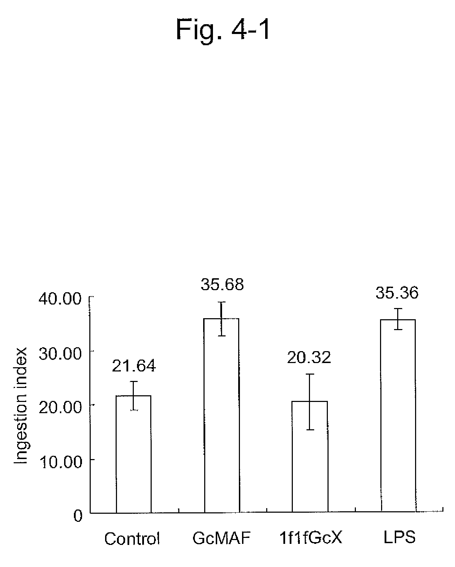

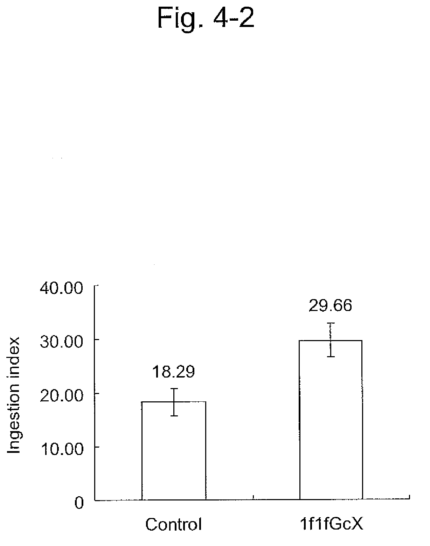

Fig. 4-1 and Fig. 4-2 show the results of Experiment 1 and Experiment 2 using purified pre-GcMAF (1f1fGcX).

In Experiment 1, the mean ingestion index (n = 3) of the group to which GcMAF had been administered increased, compared with that of the control group, although no change was observed in the group to which purified pre-GcMAF (If1fGcX) had been administered (Fig. 4-1). As a result of the t-test for determining the significant difference between the treatment groups and the control group, significant differences p of 0.0040 (p < 0.01) and p of 0.0017 (p < 0.01) were observed in the group to which GcMAF had been administered and in the group to which LPS had been administered, respectively; however, no significant difference was observed in the group to which purified pre-GcMAF (1f1fGcX) had been administered (p = 0.15959).

In Experiment 2, the mean ingestion index of macrophages (n = 3) of the group to which purified pre-GcMAF subjected to preliminary culture with non-adherent peritoneal cells (1f1fGcX) had been administered increased, compared with that of the control group (Fig. 4-2). As a result of the t-test for determining the significant difference between the treatment group and the control group, a significant difference p of 0.0009 (p < 0.01) was observed in the group to which purified pre-GcMAF subjected to preliminary culture with non-adherent peritoneal cells (1f1fGcX) had been administered. While purified pre-GcMAF (1f1fGcX) did not directly affect macrophage phagocytic activity, purified pre-GcMAF, enhanced the macrophage phagocytic activity when subjected to preliminary culture with non-adherent peritoneal cells.

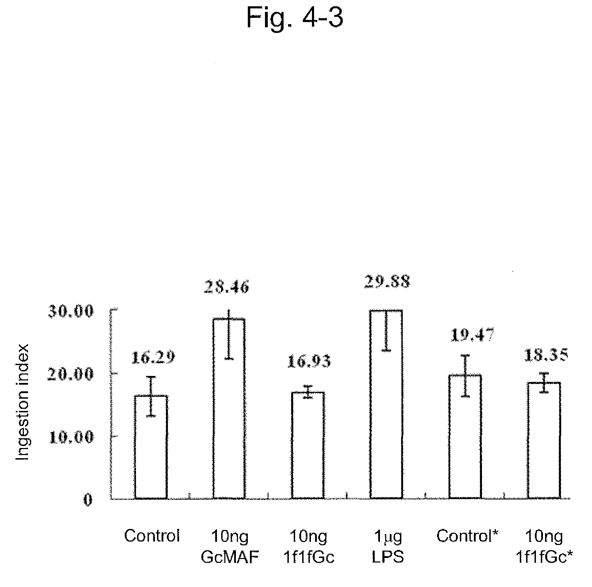

The starting material (i.e., Gc globulin of the subtype 1f1f) did not enhance macrophage phagocytic activity in either Experiment 1 or 2 (Fig. 4-3).

The above results demonstrate that purified pre-GcMAF (1f1fGcX) exhibits a macrophage phagocytic capacity similar to that of GcMAF in the presence of non-adherent peritoneal cells (i.e., *in vivo*).

### Example 3-2: Influence of purified pre-GcMAF (1s1sGcX) on macrophage phagocytic activity (in vitro)

Purified pre-GcMAF originating from Gc globulin of the subtype 1s1s was prepared in the same manner as in Example 1 (hereafter, referred to as "purified pre-GcMAF (1s1sGcX)"), and the influence of purified pre-GcMAF (1s1sGcX) on macrophage phagocytic activity was inspected in the same manner as in Experiment 1 and Experiment 2 of Example 3-1.

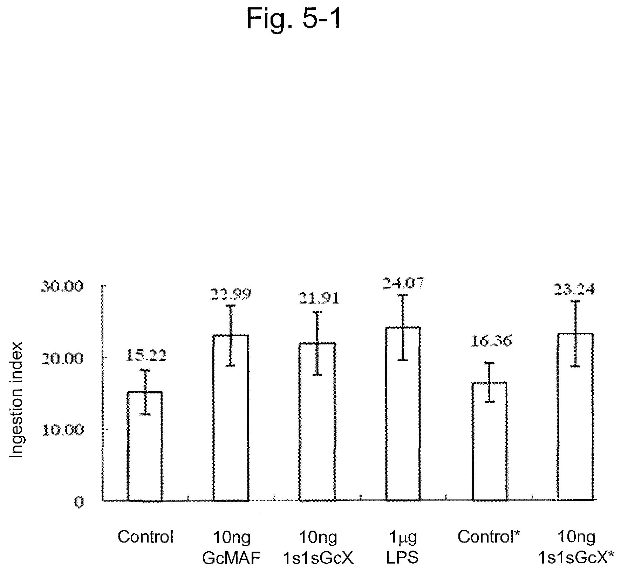

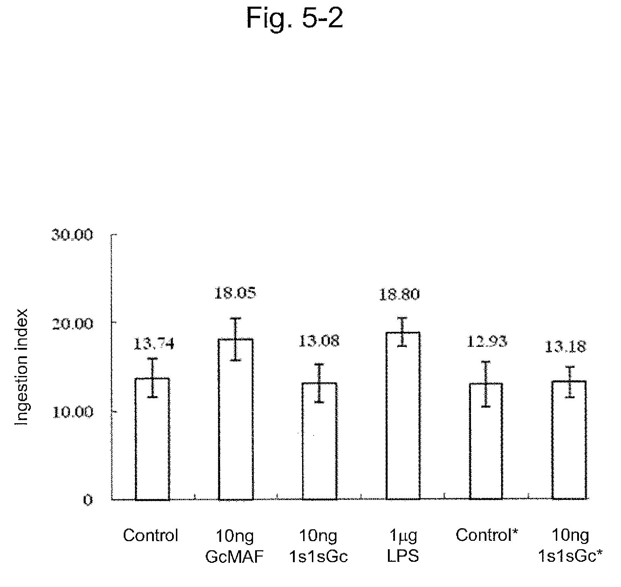

Fig. 5-1 and Fig. 5-2 show the results.

As shown in Fig. 5-1, the mean ingestion index of macrophages (n = 4) of the group to which GcMAF had been administered and of the group to which purified pre-GcMAF (1f1fGcX) had been administered was found to have increased, compared with that of the control group, as a result of Experiment 1. As a result of the t-test for determining the significant difference between the treatment groups and the control group, significant differences p of 0.0238 (p < 0.05) and p of 0.0463 (p < 0.05) were observed in the group to which GcMAF had been administered and in the group to which purified pre-GcMAF (1f1fGcX) had been administered, respectively. In Experiment 2, the mean ingestion index of macrophages (n = 4) of the group to which purified pre-GcMAF (1f1fGcX) had been administered (1s1sGcX*) increased, compared with that of the control group. As a result of the t-test for determining the significant difference between the treatment group and the control group*, a significant difference p of 0.0396 (p < 0.05) was observed in the group to which purified pre-GcMAF (1f1fGcX) had been administered (1s1sGcX*).

As shown in Fig. 5-2, the starting material (i.e., Gc globulin of the subtype 1s1s) did not enhance the phagocytic activity in either Experiment 1 or 2.

The above results demonstrate that purified pre-GcMAF (1f1fGcX) exhibits a macrophage phagocytic capacity similar to that of GcMAF. In addition, the macrophage phagocytic capacity of purified pre-GcMAF (1s1sGcX), which is similar to that of GcMAF, was found to remain at the same level in the presence of non-adherent peritoneal cells (i.e., *in vivo*).

### Example 3-3: Influence of purified pre-GcMAF (22GcX) on macrophage phagocytic activity (in vitro)

Purified pre-GcMAF originating from Gc globulin of the subtype 22 was prepared in the same manner as in Example 1 (hereafter, referred to as "purified pre-GcMAF (22GcX)"), and the influence of purified pre-GcMAF (22GcX) on the macrophage phagocytic activity was inspected in the same manner as in Experiment 1 and Experiment 2 of Example 3-1.

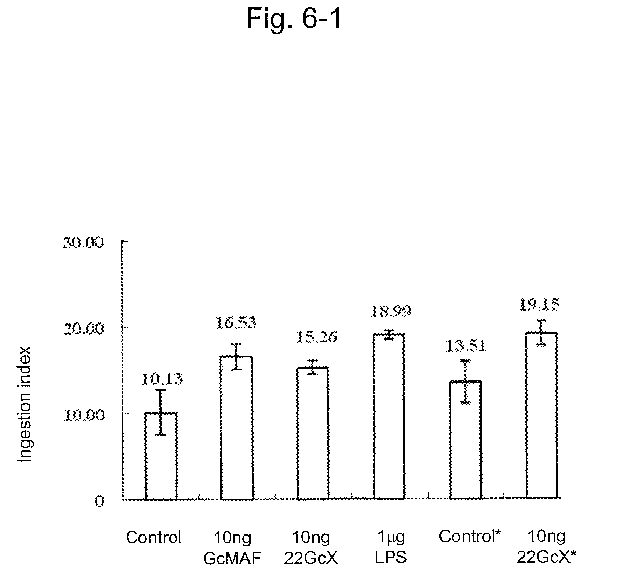

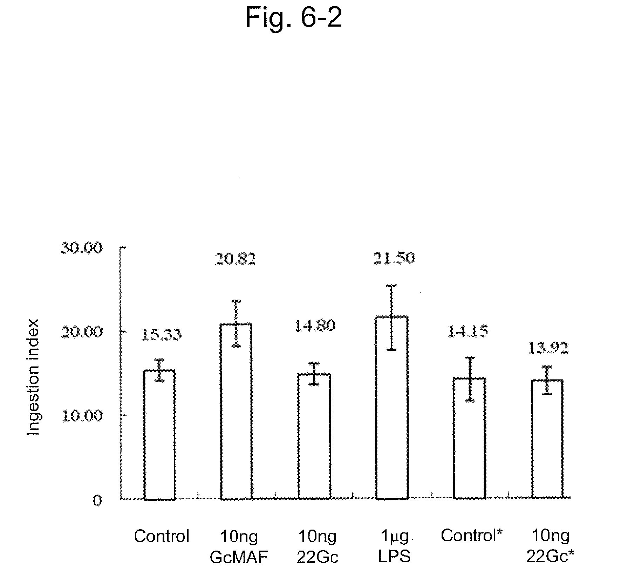

Fig. 6-1 and Fig. 6-2 show the results.

As shown in Fig. 6-1, the mean ingestion index of macrophages (n = 4) of the group to which GcMAF had been administered and of the group to which purified pre-GcMAF (22GcX) had been administered was found to have increased, compared with that of the control group, as a result of Experiment 1. As a result of the t-test for determining the significant difference between the treatment groups and the control group, significant differences p of 0.0214 (p < 0.05) and p of 0.0319 (p < 0.05) were observed in the group to which GcMAF had been administered and in the group to which purified pre-GcMAF had been administered, respectively. In Experiment 2, the mean ingestion index of macrophages (n = 4) of the group to which purified pre-GcMAF (22GcX) had been administered (22GcX*) increased, compared with that of the control group. As a result of the t-test for determining the significant difference between the treatment group and the control group*, a significant difference p of 0.0254 (p < 0.05) was observed in the group to which purified pre-GcMAF (22GcX) had been administered (22GcX*).

As shown in Fig. 6-2, the starting material (i.e., Gc globulin of the subtype 22) did not enhance the phagocytic activity in either Experiment 1 or 2.

The above results demonstrate that purified pre-GcMAF (22GcX) exhibits a macrophage phagocytic capacity similar to that of GcMAF. In addition, the macrophage phagocytic capacity of purified pre-GcMAF (22GcX), which is similar to that of GcMAF, was found to remain at the same level in the presence of non-adherent peritoneal cells (i.e., *in vivo*).

### Example 4: Activity of purified pre-GcMAF (1f1fGcX) for inhibition of angiogenesis (in vivo)

Whether or not purified pre-GcMAF would exhibit activity of angiogenesis inhibition, as with the case of GcMAF, was examined by inspecting the activity of angiogenesis inhibition *in vivo* by chicken embryo chorioallantoic membrane assays (CAM assays).

### <Experiment>

Fertilized chicken eggs on day 0 of incubation were cultured in an incubator at 37.6°C for 4 days, holes were made with a drill at two positions of each eggshell (i.e., a position in the upper part of the air chamber and a position on the lateral side of a chicken egg), and about 4 ml of egg albumen was removed by suction through a hole on the lateral side of each chicken egg. Subsequently, the yolk sak and the embryo were peeled from each shell membrane by suction through the hole in the upper part of the air chamber using a silicon dropper, and the hole on the lateral side of each chicken egg was sealed with Opsite. Subsequently, in each egg, the eggshell around the hole in the upper part of the air chamber was partially removed to expose the shell membrane, the upper part of the air chamber was covered with a stainless steel cap, and culture was conducted at 39°C for 24 hours. After culture, the size of the chorioallantoic membrane (CAM) of each egg was confirmed to be 2 to 3 mm, a silicon ring was placed at the center of each CAM, purified GcMAF (synthesized in the laboratory of Professor Hori at the Institute of Technology and Science, University of Tokushima) and purified pre-GcMAF (1f1fGcX) were administered, and culture was conducted at 39°C for 1 day and at 39.5°C for an additional 1 day. After culture, each eggshell was removed, about 1 ml of Intralipos was injected into each CAM, the number and the size of capillary blood vessels grown on each CAM were visually inspected, and the number of chicken eggs in which inhibition was observed was divided by the total number of eggs used in the experiment to determine the angiogenesis inhibition rate.

### <Results>

The angiogenesis inhibition rate for 100 ng of GcMAF *in vivo* was 20%, which was somewhat weaker than that of a positive control (i.e., TX-1934, synthesized in the laboratory of Professor Hori at the Institute of Technology and Science, University of Tokushima) (inhibition rate: 30%).

The inhibition rate attained with the use of the same dose (100 ng) of purified pre-GcMAF (1f1fGcX) was 23%, and that attained with the use of 10 ng of purified pre-GcMAF (1f1fGcX) was 31 %, which was significantly different from the control group (p < 0.05) and was somewhat higher than that of TX-1934 (inhibition rate: 25%).

The results demonstrate that the activity of purified pre-GcMAF (1f1fGcX) for angiogenesis inhibition is somewhat higher than that of GcMAF.

### Example 5-1: Simple synthesis of pre-GcMAF from serum and inspection of physical properties thereof

In order to inspect whether or not purified pre-GcMAF can be directly synthesized from the blood serum, 175 µl of 100 mM SPB and 25 µl of 10 mU/µl of β-galactosidase (from bovine liver, Grade III, Sigma, Lot No. 54H7025, G1875) were added to 50 µl of the prepared blood serum (originating from a human having the subtype 1f1f), and the resultant was then incubated at 37.5°C for 1 hour. The reaction product was transferred to a 2.0-ml Eppendorf tube containing 1.0 g of 25(OH)VD₃ (synthesized in the laboratory of Professor Hori at the Institute of Technology and Science, University of Tokushima), and the tube was subjected to shaking at 4°C for 1 hour. After centrifugation was carried out at 4°C and 13,000 rpm for 2 minutes and the supernatant was removed, the precipitate was washed with shaking with 0.5 ml of STE buffer for 60 seconds, centrifugation was carried out at 4°C and 13,000 rpm for an additional 2 minutes, and the supernatant was removed. This procedure was repeated 3 times. Thereafter, 0.5 ml of 5.0 M acetate buffer was added, the resultant was subjected to shaking for 60 seconds, the Gc globulin derivative was eluted, and centrifugation was carried out at 13,000 rpm for 2 minutes to extract the supernatant. This procedure was repeated 3 times, the eluates were combined, and the solution was exchanged with 10 mM SPB buffer using Microcon (Millipore, Lot No. R9DN95311, 10,000 MWCO). Thus, roughly purified pre-GcMAF (2) was obtained (21.64 µg/29 µl).

As a result of protein visualization via SDS-PAGE (CBB staining) of roughly purified pre-GcMAF (2), 3 bands were detected in total. As a result of Western blot analysis using human anti-Gc globulin, a positive reaction was observed only in the darkest band corresponding to Gc globulin at around a molecular weight of 56 kDa. The other two bands did not show positive reactions.

With the use of general-purpose image processing software for Windows (WinRoof), the density of the target band stained with CBB was designated as 1.0, the density and the area of the stained bands were assayed, the value attained for the target band was divided by the sum of values attained for all bands to determine the purity of the target band, and the purity was expressed in percentages. The results are shown in Table 1. The purity of the target band was 66.11%.

**Table 1**

| Number | Density | Area | Density x area |
|---|---|---|---|
| 1 | 1.0000 | 18096.48 | 18096.48 |
| 2 | 0.6084 | 14264.01 | 8678.22 |
| 3 | 0.3152 | 1904.48 | 600.29 |

| | | | |
|---|---|---|---|
| Purity of target band = 18096.48/(18096.48 + 8678.22 + 600.29) | | | |

Roughly purified products (2) obtained from the blood serum by a simple process contain pre-GcMAF, and they also contain serum-derived proteins as contaminants.

Example 5-2: Western blot analysis of roughly purified pre-GcMAF (2) using PNA lectin and HPA lectin

As a result of Western blot analysis of bands exhibiting positive reactions using PNA lectin and HPA lectin, stained bands corresponding to Gc globulin and GcMAF were not observed as in the case of Example 2 above.

As a result of Western blot analysis of roughly purified pre-GcMAF obtained by a simple process from the blood serum of a human having the subtype 1s1s or 22 in the same manner as that described above, stained bands corresponding to Gc globulin and GcMAF were not observed in pre-GcMAF originating from the subtype 1s1s. Pre-GcMAF originating from the subtype 22 was subjected to Western blot analysis using PNA lectin. As a result, a stained band corresponding to Gc globulin was not detected.

The results indicate that the roughly purified product obtained by a simple process from the blood serum of a human having the subtype 1s1s or 22 contains pre-GcMAF. Example 6: Production example of pre-GcMAF and inspection of physical properties thereof (I) Synthesis of pre-GcMAF from serum and purification of pre-GcMAF

STE buffer (1.0 ml) and 150 µl of 10 mU/µl β-galactosidase (from bovine liver, Grade III, Sigma, Lot No. 54H7025, G1875) were added to 1.0 ml of blood serum (originating from a human having the subtype 1f1f), and the resultant was incubated at 37.5°C for 1 hour.

STE buffer (10 ml) was added to the reaction solution, and the resultant was allowed to flow through an affinity column (10 mm × 80 mm) containing 25(OH)VD₃-bound beads (synthesized in the laboratory of Professor Hori at the Institute of Technology and Science, University of Tokushima) at a rate of 0.4 ml/min. Further, STE buffer was allowed to flow through the column for 5 minutes, STE buffer containing 6 M guanidine (pH 7.4) was allowed to flow at a rate of 2.0 ml/min, and about 25 ml of the eluted Gc globulin derivative was collected. The collected solution was injected into a dialysis bag with one end thereof closed with a clip, the other end thereof was closed with a clip, a float was applied thereto, the resultant was allowed to float in 4 liters of 5 mM phosphate buffer (SPB), and dialysis was then carried out. The dialysate solution was exchanged with fresh solution 90 minutes, 180 minutes, and 270 minutes after the initiation of dialysis. The third step of dialysis was carried out overnight.

### (II) Secondary purification through hydroxyapatite column and inspection of physical properties thereof

In order to remove contaminants such as reagents, the solution after dialysis was collected, and the sample was allowed to flow through a hydroxyapatite column (BioRad, Bio-Scale™ Mini CHT Type III, 40 µM Cartridge, Catalog # 732-4332, Lot No. B012409B) at a rate of 0.2 ml/min. Thereafter, 5 mM SPB buffer was allowed to flow therethrough at a rate of 2.0 ml/min to wash the column. When the baseline was stabilized, gradient elution was carried out with 200 mM SPB buffer (pH 7.4), and approximately 9 ml of a Gc globulin-containing solution was collected. The collected solution was applied to Centricon (Millipore, Lot No. R0DA20931, 30,000 MWCO), and centrifugation was carried out in a centrifuge provided with a collection cup and an angle rotor at 3,990 G for 10 minutes to obtain 200 µl of the final purified product. The protein concentration was 200.6 µg/200 µl, determined according to the BCA method.

The final purified product was confirmed to correspond to a single band via protein visualization by SDS-PAGE (CBB staining), and the band exhibited a positive reaction to Western blot analysis using human anti-globulin.

### Example 7: Tumor growth control by purified pre-GcMAF (1f1fGcX) in mice with pulmonary metastasis of LLCs (Lewis lung carcinoma cells)

5-week-old C57BL/6 mice were conditioned for at least 7 days, and 2 x 10⁵ LLCs were injected thereinto through the caudal vein. The mice were then divided into four groups at random: i.e., a control group; a positive control group (to which GcMAF was administered at 4 ng/kg/day); and treatment groups (a treatment group to which purified pre-GcMAF (1f1fGcX) was administered at 0.04 µg/kg/day and another treatment group to which purified pre-GcMAF (1f1fGcX) was administered at 0.4 µg/kg/day). The pharmaceutical agent was administered intraperitoneally (i.p.) for 10 days, mice were killed on the following day (day 11), the number of tumor nodules developed in the lungs was counted (the total number, the number of tumor nodules smaller than 2 mm, and the number of tumor nodules 2 mm or larger), and the effects of the administration of purified pre-GcMAF for inhibiting tumor growth were inspected.

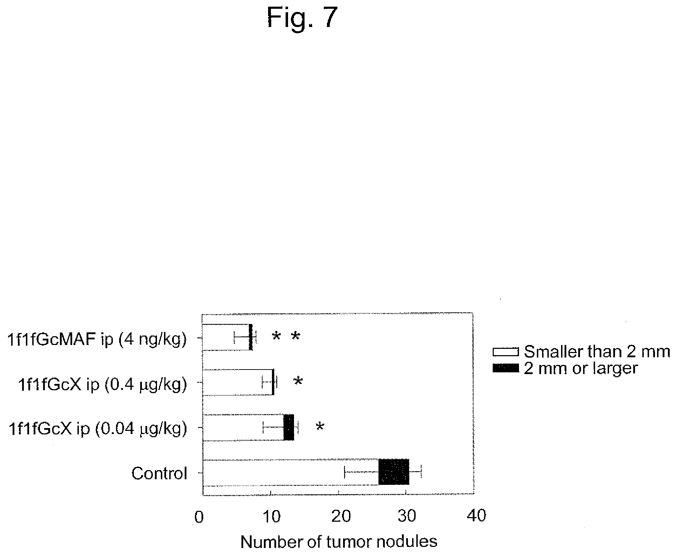

The results are shown in Fig. 7.

The number of tumor nodules developed in the lungs (mean ± SEM) of the treatment groups (n = 3 or 4) (to which purified pre-GcMAF (1f1fGcX) prepared in Example 1 had been administered) was significantly reduced compared with the figures for the control group. Such reduction in the number of tumor nodules was examined by the Bonferroni multiple comparison test. As a result, a significant difference was observed between the treatment group and the control group (* p < 0.05). This demonstrates that administration of purified pre-GcMAF (1f1fGcX) at a dose of 0.04 µg/kg or 0.4 µg/kg exerts significant anti-tumor, activity.

As shown in Fig. 7, the number of tumor nodules grown to a size of 2 mm or larger was reduced depending on the doses of purified pre-GcMAF (1f1fGcX) in the treatment groups. Thus, growth inhibition of tumor nodules was observed as a result of administration of purified pre-GcMAF (1f1fGcX). When a tumor grows to 3 mm³ or larger (tumor diameter: about 2 mm), in general, oxygen concentration is lowered in the center of the tumor, and angiogenesis is then induced. Growth inhibition of tumor nodules observed in the treatment groups indicates that purified pre-GcMAF (1f1fGcX) inhibits angiogenesis in tumor tissue and blocks tumor growth. A reduction was observed in the number of tumor nodules smaller than 2 mm in the treatment groups. This indicates that secondary spreading of cancer cells that were primarily implanted in the pulmonary tissue through the blood vessels is inhibited.

### Example 8: Tumor growth control by different types of purified pre-GcMAF in mice with pulmonary metastasis of LLC (Lewis lung carcinoma cells)

5-week-old C57BL/6 mice were conditioned for at least 7 days, and 2 x 10⁵ LLCs were injected thereinto through the caudal vein. The mice were then divided into four groups at random: i.e., a control group; and treatment groups (treatment groups to which 1f1fGcX, 1s1sGcX, and 22GcX were administered, respectively, at 0.4 µg/kg/day intraperitoneally for 10 days; purified pre-GcMAF was prepared as in Examples 1, 3-2, and 3-3). The pharmaceutical agent was administered intraperitoneally (i.p.) for 10 days, mice were killed on the following day (day 11), the number of tumor nodules developed in the lungs was counted (the total number, the number of tumor nodules smaller than 2 mm, and the number of tumor nodules 2 mm or larger), and the effects of purified pre-GcMAF for inhibiting tumor growth were inspected.

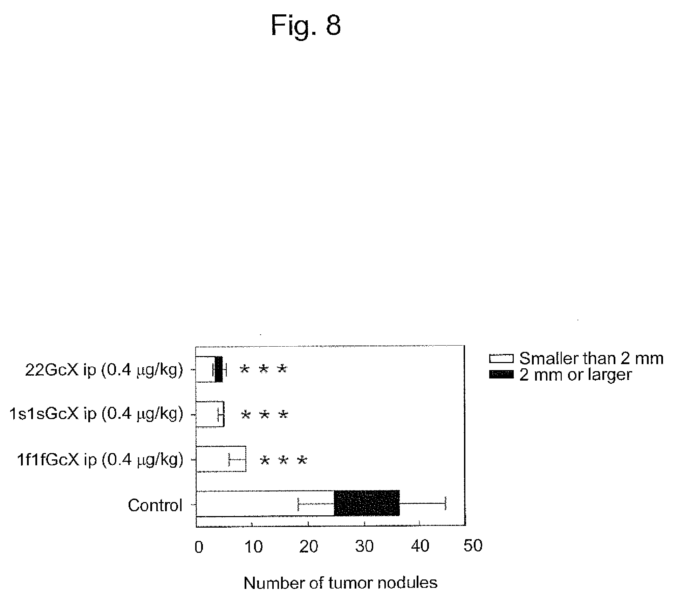

The results are shown in Fig. 8. The results are shown in terms of the average number of tumor nodules developed in the lungs ± SEM of each group (n = 3 or 4).

The average number of tumor nodules of the treatment groups was observed to be significantly reduced compared with the figures for the control group. As a result of the Bonferroni multiple comparison test for comparing the treatment groups with the control group, reduction in the number of tumor nodules was observed to a significant extent upon administration of purified pre-GcMAF (1f1fGcX), purified pre-GcMAF (1s1sGcX), and purified pre-GcMAF (22GcX) (*** p < 0.001). Such results exhibit a similar trend as the results of evaluation of the macrophage activating capacity of purified pre-GcMAFs described in Examples 1 to 3-3.

The number of the tumor nodules grown to 2 mm or larger was smaller when purified pre-GcMAF was administered, compared with the number thereof when lung cancer was not treated. No tumor nodules were developed when purified pre-GcMAF (1f1fGcX) and purified pre-GcMAF (1s1sGcX) were administered, and one tumor nodule was developed when purified pre-GcMAF (22GcX) was administered. The results indicate that these purified pre-GcMAFs (1f1fGcX, 1s1sGcX, and 22GcX) inhibit angiogenesis in tumor tissue and inhibit tumor growth.

### Industrial Applicability

The present invention can provide degalactosylated Gc globulin that can be easily produced and used as GcMAF, regardless of Gc globulin subtype. As with the case of GcMAF, accordingly, degalactosylated Gc globulin can be administered to a patient for the purpose of treatment or prevention of a variety of diseases or disorders through, for example, activation of macrophages, inhibition of angiogenesis, and reinforcement of the resistance or healing ability of a patient.

## Claims

1. A pharmaceutical composition comprising one or a plurality of human degalactosylated Gc globulins for use in healing of wounds or treating allergic diseases, autoallergic diseases, adverse reactions caused by therapeutic agents or angiogenic diseases, said one or a plurality of human degalactosylated Gc globulins being selected from the group consisting of (i) to (iii) below:
(i) the degalactosylated Gc globulin which is obtained by a treatment of Gc globulin of subtype 1f and/or subtype 1s derived from human blood plasma or serum with only β-galactosidase;
(ii) a human degalactosylated Gc globulin comprising the amino acid sequence as shown in SEQ ID NO: 1 and having a sugar chain structure in which N-acetylgalactosamine is bound to threonine at either position 418 or 420 of the amino acid sequence as shown in SEQ ID NO: 1 and sialic acid is further bound to N-acetylgalactosamine; and
(iii) a human degalactosylated Gc globulin comprising the amino acid sequence as shown in SEQ ID NO: 2 and having a sugar chain structure in which N-acetylgalactosamine is bound to threonine at either position 418 or 420 of the amino acid sequence as shown in SEQ ID NO: 2 and α-mannose is further bound to N-acetylgalactosamine.

2. The pharmaceutical composition for use according to claim 1, which further comprises one or a plurality of human degalactosylated Gc globulins selected from the group consisting the following:
the degalactosylated Gc globulin which is obtained by a treatment of Gc globulin of subtype 2 derived from human blood plasma or serum with only β-galactosidase; and
a degalactosylated Gc globulin comprising the amino acid sequence as shown in SEQ ID NO: 3 and having a sugar chain structure in which N-acetylgalactosamine is bound to threonine at position 418 of the amino acid sequence as shown in SEQ ID NO: 3.

3. A pharmaceutical composition comprising one or a plurality of human degalactosylated Gc globulins for use in treating cancer, said one or a plurality of human degalactosylated Gc globulins being selected from the group consisting of (i) to (iii) below:
(i) the degalactosylated Gc globulin which is obtained by a treatment of Gc globulin of subtype 1f and/or subtype 1s derived from human blood plasma or serum with only β-galactosidase;
(ii) a human degalactosylated Gc globulin comprising the amino acid sequence as shown in SEQ ID NO: 1 and having a sugar chain structure in which N-acetylgalactosamine is bound to threonine at either position 418 or 420 of the amino acid sequence as shown in SEQ ID NO: 1 and sialic acid is further bound to N-acetylgalactosamine; and
(iii) a human degalactosylated Gc globulin comprising the amino acid sequence as shown in SEQ ID NO: 2 and having a sugar chain structure in which N-acetylgalactosamine is bound to threonine at either position 418 or 420 of the amino acid sequence as shown in SEQ ID NO: 2 and α-mannose is further bound to N-acetylgalactosamine.

4. The pharmaceutical composition for use according to claim 3, which further comprises one or a plurality of human degalactosylated Gc globulins selected from the group consisting the following:
the degalactosylated Gc globulin which is obtained by a treatment of Gc globulin of subtype 2 derived from human blood plasma or serum with only β-galactosidase; and
a degalactosylated Gc globulin comprising the amino acid sequence as shown in SEQ ID NO: 3 and having a sugar chain structure in which N-acetylgalactosamine is bound to threonine at position 418 of the amino acid sequence as shown in SEQ ID NO: 3.

5. The pharmaceutical composition for use according to any one of claims 1 to 4, wherein the human degalactosylated Gc globulin is prepared from human Gc globulin originating from blood plasma or serum of a subject to which the pharmaceutical composition is administered.

6. A preparation comprising one or a plurality of degalactosylated Gc globulins for use in treating of cancer of a patient selected from the group consisting of (i) to (iii) below:
(i) the degalactosylated Gc globulin which is obtained by a treatment of Gc globulin of subtype 1f and/or subtype 1s derived from human blood plasma or serum with only β-galactosidase;
(ii) a degalactosylated Gc globulin comprising the amino acid sequence as shown in SEQ ID NO: 1 and having a sugar chain structure in which N-acetylgalactosamine is bound to threonine at either position 418 or 420 of the amino acid sequence as shown in SEQ ID NO: 1 and sialic acid is further bound to N-acetylgalactosamine; and
(iii) a degalactosylated Gc globulin comprising the amino acid sequence as shown in SEQ ID NO: 2 and having a sugar chain structure in which N-acetylgalactosamine is bound to threonine at either position 418 or 420 of the amino acid sequence as shown in SEQ ID NO: 2 and α-mannose is further bound to N-acetylgalactosamine.

7. The preparation for use according to claim 6, wherein the degalactosylated GcG is prepared from Gc globulin originating from blood plasma or serum of a cancer patient to which the degalactosylated GcG is administered.

8. The preparation for use according to claim 6 or 7, which further comprises one or a plurality of human degalactosylated Gc globulins selected from the group consisting of the following:
the degalactosylated Gc globulin which is obtained by a treatment of Gc globulin of subtype 2 derived from human blood plasma or serum with only β-galactosidase; and
a degalactosylated Gc globulin comprising the amino acid sequence as shown in SEQ ID NO: 3 and having a sugar chain structure in which N-acetylgalactosamine is bound to threonine at position 418 of the amino acid sequence as shown in SEQ ID NO: 3.

9. A preparation comprising one or a plurality of human degalactosylated Gc globulins selected from the group consisting of (i) to (iii) below for use in healing of wound or treating allergic diseases, autoallergic diseases, adverse reactions caused by therapeutic agents or angiogenic diseases:
(i) the degalactosylated Gc globulin which is obtained by a treatment of Gc globulin of subtype 1f and/or subtype 1s derived from human blood plasma or serum with only β-galactosidase;
(ii) a human degalactosylated Gc globulin comprising the amino acid sequence as shown in SEQ ID NO: 1 and having a sugar chain structure in which N-acetylgalactosamine is bound to threonine at either position 418 or 420 of the amino acid sequence as shown in SEQ ID NO: 1 and sialic acid is further bound to N-acetylgalactosamine; and
(iii) a human degalactosylated Gc globulin comprising the amino acid sequence as shown in SEQ ID NO: 2 and having a sugar chain structure in which N-acetylgalactosamine is bound to threonine at either position 418 or 420 of the amino acid sequence as shown in SEQ ID NO: 2 and α-mannose is further bound to N-acetylgalactosamine.

10. The preparation for use according to claim 9, which further comprises one or a plurality of human degalactosylated Gc globulins selected from the group consisting the following:
the degalactosylated Gc globulin which is obtained by a treatment of Gc globulin of subtype 2 derived from human blood plasma or serum with only β-galactosidase; and
a degalactosylated Gc globulin comprising the amino acid sequence as shown in SEQ ID NO: 3 and having a sugar chain structure in which N-acetylgalactosamine is bound to threonine at position 418 of the amino acid sequence as shown in SEQ ID NO: 3.

## Patentansprüche

1. Pharmazeutische Zusammensetzung, die ein oder mehrere degalactosylierte Gc-Globuline des Menschen umfasst, zur Verwendung bei der Wundheilung oder der Behandlung allergischer Erkrankungen, autoallergischer Erkrankungen, Unverträglichkeitsreaktionen, die durch therapeutische Mittel verursacht werden, oder angiogener Erkrankungen, wobei das eine oder die mehreren degalactosylierte (n) Gc-Globulin(e) des Menschen aus der Gruppe ausgewählt ist/sind, welche aus den nachstehenden (i) bis (iii) besteht:
(i) dem degalactosylierten Gc-Globulin, wie es erhalten wird, indem ein Gc-Globulin des Subtyps 1f und/oder des Subtyps 1s, welches aus dem Blutplasma oder Serum des Menschen stammt, ausschließlich mit β-Galactosidase behandelt wird;
(ii) einem degalactosylierten Gc-Globulin des Menschen, das die Aminosäuresequenz gemäß der Darstellung in der SEQ-ID Nr. 1 umfasst und eine Zuckerkettenstruktur aufweist, in welcher N-Acetylgalactosamin an Position 418 oder 420 der Aminosäuresequenz gemäß der Darstellung in SEQ-ID Nr. 1 an Threonin gebunden ist und weiterhin Sialinsäure an N-Acetylgalactosamin gebunden ist; und
(iii) einem degalactosylierten Gc-Globulin des Menschen, das die Aminosäuresequenz gemäß der Darstellung in der SEQ-ID Nr. 2 umfasst und eine Zuckerkettenstruktur aufweist, in welcher N-Acetylgalactosamin an Position 418 oder 420 der Aminosäuresequenz gemäß der Darstellung in SEQ-ID Nr. 2 an Threonin gebunden ist und weiterhin α-Mannose an N-Acetylgalactosamin gebunden ist.

2. Pharmazeutische Zusammensetzung zur Verwendung gemäß Anspruch 1, welche weiterhin ein oder mehrere degalactosylierte Gc-Globuline des Menschen umfasst, die aus der Gruppe ausgewählt sind, welche aus den folgenden besteht:
dem degalactosylierte Gc-Globulin, wie es erhalten wird, indem ein Gc-Globulin des Subtyps 2, welches aus dem Blutplasma oder Serum des Menschen stammt, ausschließlich mit β-Galactosidase behandelt wird; und
einem degalactosylierten Gc-Globulin, das die Aminosäuresequenz gemäß der Darstellung in der SEQ-ID Nr. 3 umfasst und eine Zuckerkettenstruktur aufweist, in welcher N-Acetylgalactosamin an Position 418 der Aminosäuresequenz gemäß der Darstellung in SEQ-ID Nr. 3 an Threonin gebunden ist.

3. Pharmazeutische Zusammensetzung, die ein oder mehrere degalactosylierte Gc-Globuline des Menschen umfasst, zur Verwendung bei der Behandlung von Krebs, wobei das eine oder die mehreren degalactosylierte(n) Gc-Globulin(e) des Menschen aus der Gruppe ausgewählt ist/sind, welche aus den nachstehenden (i) bis (iii) besteht:
(i) dem degalactosylierten Gc-Globulin, wie es erhalten wird, indem ein Gc-Globulin des Subtyps 1f und/oder des Subtyps 1s, welches aus dem Blutplasma oder Serum des Menschen stammt, ausschließlich mit β-Galactosidase behandelt wird;
(ii) einem degalactosylierten Gc-Globulin des Menschen, das die Aminosäuresequenz gemäß der Darstellung in der SEQ-ID Nr. 1 umfasst und eine Zuckerkettenstruktur aufweist, in welcher N-Acetylgalactosamin an Position 418 oder 420 der Aminosäuresequenz gemäß der Darstellung in SEQ-ID Nr. 1 an Threonin gebunden ist und weiterhin Sialinsäure an N-Acetylgalactosamin gebunden ist; und
(iii) einem degalactosylierten Gc-Globulin des Menschen, das die Aminosäuresequenz gemäß der Darstellung in der SEQ-ID Nr. 2 umfasst und eine Zuckerkettenstruktur aufweist, in welcher N-Acetylgalactosamin an Position 418 oder 420 der Aminosäuresequenz gemäß der Darstellung in SEQ-ID Nr. 2 an Threonin gebunden ist und weiterhin α-Mannose an N-Acetylgalactosamin gebunden ist.

4. Pharmazeutische Zusammensetzung zur Verwendung gemäß Anspruch 3, welche weiterhin ein oder mehrere degalactosylierte Gc-Globuline des Menschen umfasst, die aus der Gruppe ausgewählt sind, welche aus den folgenden besteht:
dem degalactosylierten Gc-Globulin, wie es erhalten wird, indem ein Gc-Globulin des Subtyps 2, welches aus dem Blutplasma oder Serum des Menschen stammt, ausschließlich mit β-Galactosidase behandelt wird; und
einem degalactosylierten Gc-Globulin, das die Aminosäuresequenz gemäß der Darstellung in der SEQ-ID Nr. 3 umfasst und eine Zuckerkettenstruktur aufweist, in welcher N-Acetylgalactosamin an Position 418 der Aminosäuresequenz gemäß der Darstellung in SEQ-ID Nr. 3 an Threonin gebunden ist.

5. Pharmazeutische Zusammensetzung zur Verwendung gemäß einem beliebigen der Ansprüche 1 bis 4, wobei das degalactosylierte Gc-Globulin des Menschen ausgehend von einem Gc-Globulin des Menschen hergestellt wird, welches aus dem Blutplasma oder Serum einer Person stammt, der die pharmazeutische Zusammensetzung verabreicht werden soll.

6. Zubereitung, die ein oder mehrere der degalactosylierten Gc-Globuline umfasst, welche zur Verwendung bei der Behandlung von Krebs bei Patienten bestimmt sind, wobei sie aus der Gruppe ausgewählt sind, welche aus den nachstehenden (i) bis (iii) besteht:
(i) dem degalactosylierten Gc-Globulin, wie es erhalten wird, indem ein Gc-Globulin des Subtyps 1f und/oder des Subtyps 1s, welches aus dem Blutplasma oder Serum des Menschen stammt, ausschließlich mit β-Galactosidase behandelt wird;
(ii) einem degalactosylierten Gc-Globulin, das die Aminosäuresequenz gemäß der Darstellung in der SEQ-ID Nr. 1 umfasst und eine Zuckerkettenstruktur aufweist, in welcher N-Acetylgalactosamin an Position 418 oder 420 der Aminosäuresequenz gemäß der Darstellung in SEQ-ID Nr. 1 an Threonin gebunden ist und weiterhin Sialinsäure an N-Acetylgalactosamin gebunden ist; und
(iii) einem degalactosylierten Gc-Globulin, das die Aminosäuresequenz gemäß der Darstellung in der SEQ-ID Nr. 2 umfasst und eine Zuckerkettenstruktur aufweist, in welcher N-Acetylgalactosamin an Position 418 oder 420 der Aminosäuresequenz gemäß der Darstellung in SEQ-ID Nr. 2 an Threonin gebunden ist und weiterhin α-Mannose an N-Acetylgalactosamin gebunden ist.

7. Zubereitung zur Verwendung gemäß Anspruch 6, wobei das degalactosylierte GcG ausgehend von einem Gc-Globulin hergestellt wird, welches aus dem Blutplasma oder Serum eines Krebspatienten stammt, dem das degalactosylierte GcG verabreicht wird.

8. Zubereitung zur Verwendung gemäß Anspruch 6 oder 7, welche weiterhin ein oder mehrere der degalactosylierten Gc-Globuline des Menschen umfasst, die aus der Gruppe ausgewählt sind, welche aus den folgenden besteht:
dem degalactosylierten Gc-Globulin, wie es erhalten wird, indem ein Gc-Globulin des Subtyps 2, welches aus dem Blutplasma oder Serum des Menschen stammt, ausschließlich mit β-Galactosidase behandelt wird; und
einem degalactosylierten Gc-Globulin, das die Aminosäuresequenz gemäß der Darstellung in der SEQ-ID Nr. 3 umfasst und eine Zuckerkettenstruktur aufweist, in welcher N-Acetylgalactosamin an Position 418 der Aminosäuresequenz gemäß der Darstellung in SEQ-ID Nr. 3 an Threonin gebunden ist.

9. Zubereitung, die ein oder mehrere degalactosylierte Gc-Globuline des Menschen umfasst, welche aus der Gruppe ausgewählt sind, die aus den nachstehenden (i) bis (iii) besteht, zur Verwendung bei der Wundheilung oder der Behandlung allergischer Erkrankungen, autoallergischer Erkrankungen, Unverträglichkeitsreaktionen, die durch therapeutische Mittel verursacht werden, oder angiogener Erkrankungen:
(i) dem degalactosylierten Gc-Globulin, wie es erhalten wird, indem ein Gc-Globulin des Subtyps 1f und/oder des Subtyps 1s, welches aus dem Blutplasma oder Serum des Menschen stammt, ausschließlich mit β-Galactosidase behandelt wird;
(ii) einem degalactosylierten Gc-Globulin des Menschen, das die Aminosäuresequenz gemäß der Darstellung in der SEQ-ID Nr. 1 umfasst und eine Zuckerkettenstruktur aufweist, in welcher N-Acetylgalactosamin an Position 418 oder 420 der Aminosäuresequenz gemäß der Darstellung in SEQ-ID Nr. 1 an Threonin gebunden ist und weiterhin Sialinsäure an N-Acetylgalactosamin gebunden ist; und
(iii) einem degalactosylierten Gc-Globulin des Menschen, das die Aminosäuresequenz gemäß der Darstellung in der SEQ-ID Nr. 2 umfasst und eine Zuckerkettenstruktur aufweist, in welcher N-Acetylgalactosamin an Position 418 oder 420 der Aminosäuresequenz gemäß der Darstellung in SEQ-ID Nr. 2 an Threonin gebunden ist und weiterhin α-Mannose an N-Acetylgalactosamin gebunden ist.

10. Zubereitung zur Verwendung gemäß Anspruch 9, welche weiterhin ein oder mehrere der degalactosylierten Gc-Globuline des Menschen umfasst, die aus der Gruppe ausgewählt sind, welche aus den folgenden besteht:
dem degalactosylierten Gc-Globulin, wie es erhalten wird, indem ein Gc-Globulin des Subtyps 2, welches aus dem Blutplasma oder Serum des Menschen stammt, ausschließlich mit β-Galactosidase behandelt wird; und
einem degalactosylierten Gc-Globulin, das die Aminosäuresequenz gemäß der Darstellung in der SEQ-ID Nr. 3 umfasst und eine Zuckerkettenstruktur aufweist, in welcher N-Acetylgalactosamin an Position 418 der Aminosäuresequenz gemäß der Darstellung in SEQ-ID Nr. 3 an Threonin gebunden ist.

## Revendications

1. Composition pharmaceutique comprenant une ou une pluralité de globulines Gc dégalactosylées humaines pour une utilisation dans la cicatrisation de plaies ou le traitement de maladies allergiques, de maladies auto-allergiques, de réactions indésirables provoquées par des agents thérapeutiques ou des maladies angiogéniques, ladite une ou ladite pluralité de globulines Gc dégalactosylées humaines étant choisies dans le groupe consistant en (i) à (iii) ci-dessous :
(i) la globuline Gc dégalactosylée qui est obtenue par un traitement, avec seulement la β-galactosidase, de la globuline Gc du sous-type 1f et/ou du sous-type 1s qui dérive du plasma ou du sérum sanguin humain ;
(ii) une globuline Gc dégalactosylée humaine comprenant la séquence d'acides aminés telle que présentée dans SEQ ID N0:1 et ayant une structure de chaîne sucre dans laquelle la N-acétylgalactosamine est liée à la thréonine sur l'une ou l'autre des positions 418 ou 420 de la séquence d'acides aminés telle que présentée dans SEQ ID NO:1, et l'acide sialique est en outre lié à la N-acétylgalactosamine ; et
(iii) une globuline Gc dégalactosylée humaine comprenant la séquence d'acides aminés telle que présentée dans SEQ ID NO:2 et ayant une structure de chaîne sucre dans laquelle la N-acétylgalactosamine est liée à la thréonine sur l'une ou l'autre des positions 418 ou 420 de la séquence d'acides aminés telle que présentée dans SEQ ID NO:2, et de l'α-mannose est en outre liée à la N-acétylgalactosamine.

2. Composition pharmaceutique pour l'utilisation selon la revendication 1, qui comprend en outre une ou une pluralité de globulines Gc dégalactosylées humaines choisies dans le groupe consistant en ce qui suit :
la globuline Gc dégalactosylée qui est obtenue par un traitement, avec seulement la β-galactosidase, de la globuline Gc du sous-type 2 qui dérive du plasma ou du sérum sanguin humain ; et
une globuline Gc dégalactosylée comprenant la séquence d'acides aminés telle que présentée dans SEQ ID NO:3 et ayant une structure de chaîne sucre dans laquelle la N-acétylgalactosamine est liée à la thréonine en position 418 de la séquence d'acides aminés telle que présentée dans SEQ ID NO:3.

3. Composition pharmaceutique comprenant une ou une pluralité de globulines Gc dégalactosylées humaines pour une utilisation dans le traitement du cancer, ladite une ou ladite pluralité de globulines Gc dégalactosylées humaines étant choisies dans le groupe consistant en (i) à (iii) ci-dessous :
(i) la globuline Gc dégalactosylée qui est obtenue par un traitement, avec seulement la β-galactosidase, de la globuline Gc du sous-type 1f et/ou du sous-type 1s qui dérive du plasma ou du sérum sanguin humain ;
(ii) une globuline Gc dégalactosylée humaine comprenant la séquence d'acides aminés telle que présentée dans SEQ ID N0:1 et ayant une structure de chaîne sucre dans laquelle la N-acétylgalactosamine est liée à la thréonine sur l'une ou l'autre des positions 418 ou 420 de la séquence d'acides aminés telle que présentée dans SEQ ID NO:1, et l'acide sialique est en outre lié à la N-acétylgalactosamine ; et
(iii) une globuline Gc dégalactosylée humaine comprenant la séquence d'acides aminés telle que présentée dans SEQ ID NO:2 et ayant une structure de chaîne sucre dans laquelle la N-acétylgalactosamine est liée à la thréonine sur l'une ou l'autre des positions 418 ou 420 de la séquence d'acides aminés telle que présentée dans SEQ ID NO:2, et de l'α-mannose est en outre liée à la N-acétylgalactosamine.

4. Composition pharmaceutique pour l'utilisation selon la revendication 3, qui comprend en outre une ou une pluralité de globulines Gc dégalactosylées humaines choisies dans le groupe consistant en ce qui suit :
la globuline Gc dégalactosylée qui est obtenue par un traitement, avec seulement la β-galactosidase, de la globuline Gc du sous-type 2 qui dérive du plasma ou du sérum sanguin humain ; et
une globuline Gc dégalactosylée comprenant la séquence d'acides aminés telle que présentée dans SEQ ID NO:3 et ayant une structure de chaîne sucre dans laquelle la N-acétylgalactosamine est liée à la thréonine en position 418 de la séquence d'acides aminés telle que présentée dans SEQ ID NO:3.

5. Composition pharmaceutique pour l'utilisation selon l'une quelconque des revendications 1 à 4, dans laquelle la globuline Gc dégalactosylée humaine est préparée à partir d'une globuline Gc humaine provenant du plasma ou du sérum sanguin d'un sujet auquel la composition pharmaceutique est administrée.

6. Préparation comprenant une ou une pluralité de globulines Gc dégalactosylées, pour une utilisation dans le traitement du cancer d'un patient, choisies dans le groupe consistant en (i) à (iii) ci-dessous :
(i) la globuline Gc dégalactosylée qui est obtenue par un traitement, avec seulement la β-galactosidase, de la globuline Gc du sous-type 1f et/ou du sous-type 1s qui dérive du plasma ou du sérum sanguin humain ;
(ii) une globuline Gc dégalactosylée humaine comprenant la séquence d'acides aminés telle que présentée dans SEQ ID N0:1 et ayant une structure de chaîne sucre dans laquelle la N-acétylgalactosamine est liée à la thréonine sur l'une ou l'autre des positions 418 ou 420 de la séquence d'acides aminés telle que présentée dans SEQ ID NO:1, et l'acide sialique est en outre lié à la N-acétylgalactosamine ; et
(iii) une globuline Gc dégalactosylée humaine comprenant la séquence d'acides aminés telle que présentée dans SEQ ID NO:2 et ayant une structure de chaîne sucre dans laquelle la N-acétylgalactosamine est liée à la thréonine sur l'une ou l'autre des positions 418 ou 420 de la séquence d'acides aminés telle que présentée dans SEQ ID NO:2, et de l'α-mannose est en outre liée à la N-acétylgalactosamine.

7. Préparation pour l'utilisation selon la revendication 6, dans laquelle la GcG dégalactosylée est préparée à partir d'une globuline Gc provenant du plasma ou du sérum sanguin d'un patient cancéreux auquel la GcG dégalactosylée est administrée.

8. Préparation pour l'utilisation selon la revendication 6 ou 7, qui comprend en outre une ou une pluralité de globulines Gc dégalactosylées humaines choisies dans le groupe consistant en ce qui suit :
la globuline Gc dégalactosylée qui est obtenue par un traitement, avec seulement la β-galactosidase, de la globuline Gc du sous-type 2 qui dérive du plasma ou du sérum sanguin humain ; et
une globuline Gc dégalactosylée comprenant la séquence d'acides aminés telle que présentée dans SEQ ID NO:3 et ayant une structure de chaîne sucre dans laquelle la N-acétylgalactosamine est liée à la thréonine en position 418 de la séquence d'acides aminés telle que présentée dans SEQ ID NO:3.

9. Préparation selon l'une ou une pluralité de globulines Gc dégalactosylées humaines du groupe consistant en (i) à (iii) ci-dessous pour une utilisation dans la cicatrisation d'une plaie ou le traitement de maladies allergiques, de maladies auto-allergiques, de réactions indésirables provoquées par des agents thérapeutique, ou de maladies angiogéniques :
(i) la globuline Gc dégalactosylée qui est obtenue par un traitement, avec seulement la β-galactosidase, de la globuline Gc du sous-type 1f et/ou du sous-type 1s qui dérive du plasma ou du sérum sanguin humain ;
(ii) une globuline Gc dégalactosylée humaine comprenant la séquence d'acides aminés telle que présentée dans SEQ ID NO:1 et ayant une structure de chaîne sucre dans laquelle la N-acétylgalactosamine est liée à la thréonine sur l'une ou l'autre des positions 418 ou 420 de la séquence d'acides aminés telle que présentée dans SEQ ID NO:1, et l'acide sialique est en outre lié à la N-acétylgalactosamine ; et
(iii) une globuline Gc dégalactosylée humaine comprenant la séquence d'acides aminés telle que présentée dans SEQ ID NO:2 et ayant une structure de chaîne sucre dans laquelle la N-acétylgalactosamine est liée à la thréonine sur l'une ou l'autre des positions 418 ou 420 de la séquence d'acides aminés telle que présentée dans SEQ ID NO:2, et de l'α-mannose est en outre liée à la N-acétylgalactosamine.

10. Préparation pour l'utilisation selon la revendication 9, qui comprend en outre une ou une pluralité de globulines Gc dégalactosylées humaines choisies dans le groupe consistant en ce qui suit :
la globuline Gc dégalactosylée qui est obtenue par un traitement, avec seulement la β-galactosidase, de la globuline Gc du sous-type 2 qui dérive du plasma ou du sérum sanguin humain ; et
une globuline Gc dégalactosylée comprenant la séquence d'acides aminés telle que présentée dans SEQ ID NO:3 et ayant une structure de chaîne sucre dans laquelle la N-acétylgalactosamine est liée à la thréonine en position 418 de la séquence d'acides aminés telle que présentée dans SEQ ID NO:3.
